(19) Europäisches Patentamt

European Patent Office

Office européen des brevets

(11) **EP 2 445 394 B1**

## (12) EUROPEAN PATENT SPECIFICATION

(45) Date of publication and mention
of the grant of the patent:
**18.09.2019 Bulletin 2019/38**

(21) Application number: **10734905.2**

(22) Date of filing: **28.06.2010**

(51) Int Cl.:
***A61B 5/00*** *(2006.01)*

(86) International application number:
**PCT/EP2010/003929**

(87) International publication number:
**WO 2010/149392 (29.12.2010 Gazette 2010/52)**

(54) **METHOD AND SYSTEMT FOR PROVIDING BOTH AN ESTIMATED TRUE MEAN BLOOD GLUCOSE VALUE AND ESTIMATED GLYCATED HEMOGLOBIN (HBA1C) VALUE FROM STRUCTURED SPOT MEASUREMENTS OF BLOOD GLUCOSE**

VERFAHREN UND SYSTEM ZUR BEREITSTELLUNG EINES GESCHÄTZTEN WAHREN BLUTGLUKOSEMITTELWERTES UND EINES GESCHÄTZTEN GHB-WERTES (HBA1C) AUS STRUKTURIERTEN SPOT-BLUTGLUKOSEMESSUNGEN

PROCÉDÉ ET SYSTÈME FOURNISSANT À LA FOIS UN TAUX DE GLYCÉMIE MOYEN RÉEL ESTIMÉ ET UN TAUX D'HÉMOGLOBINE GLYCOSYLÉE (HbA1C) ESTIMÉ À PARTIR DE MESURES PONCTUELLES STRUCTURÉES DE LA GLYCÉMIE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **26.06.2009 US 492667**

(43) Date of publication of application:
**02.05.2012 Bulletin 2012/18**

(73) Proprietors:
• **Roche Diabetes Care GmbH
68305 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR HR HU IE IS IT LI LT LU LV MC MK MT NL NO PL PT RO SE SI SK SM TR**

(72) Inventors:
• **THUKRAL, Ajay
Indianapolis, IN 46256 (US)**
• **WEINERT, Stefan
Pendleton, IN 46064 (US)**
• **PAN, Zhengzheng
Fishers, IN 46037 (US)**
• **AMANN-ZALAN, Ildiko
69469 Weinheim (DE)**
• **SCHWEITZER, Matthias, Axel
65189 Wiesbaden (DE)**

(56) References cited:
• **Rohlfing CL, et al.: "Defining the Relationship Between Plasma Glucose and HbA1c" Diabetes Care vol. 25, no. 2, February 2002 (2002-02), pages 275-278, XP002607410 DOI: 10.2337/diacare.25.2.275 Retrieved from the Internet: URL:http://care.diabetesjournals.org/content/25/2/275.full.pdf+html [retrieved on 2010-10-28]**
• **McCarter RJ, et al.: "Mean Blood Glucose and Biological Variation Have Greater Influence on HbA1c Levels Than Glucose Instability: An analysis of data from the Diabetes Control and Complications Trial" Diabetes Care vol. 29, no. 2, February 2006 (2006-02), pages 352-355, XP002607411 DOI: doi:10.2337/diacare.29.02.06.dc05-1594 Retrieved from the Internet: URL:http://care.diabetesjournals.org/content/29/2/352.full.pdf+html [retrieved on 2010-10-28]**

• Shah J, et al.: "Weighted influence of glucose levels at different timesof the day on HbA1c in insulin-treated type 2 diabeticpatients. The Diabetes Outcomes in Veterans Study" Diabetic Medicine vol. 23, no. Suppl. 4, A1170, December 2006 (2006-12), page 416, XP002607412 Retrieved from the Internet: URL:http://onlinelibrary.wiley.com/doi/10. 1111/j.1464-5491.2006.02038_3.x/pdf [retrieved on 2010-10-28]

**Description**

**Technical Field**

The invention relates to physiological monitoring, and in particular to a method and system for providing both an estimated true mean blood glucose value and an estimated glycated hemoglobin (HbA1C) value from structured spot measurements of blood glucose. The invention further relates to a computer program for implementing the method for providing both an estimated true mean blood glucose value and estimated glycated hemoglobin (HbA1C) value from structured spot measurements of blood glucose.

**Background**

For monitoring glycemia, the American Diabetes Association (ADA) recommends the hemoglobin A1C test, hereinafter referred to HbA1C. Health care providers (HCPs) use HbA1C as a surrogate marker to evaluate a patient's glycemia over a previous 2 to 3 month period and as a target parameter by which to treat patients. For example, the HbA1C value, which is presented as a percentage of glycated hemoglobin, is needed by the HCP while deciding or recommending a change to a patient's therapy. Therapy modification may include a change to, an addition to, or a switch in insulin therapy, oral medication, nutrition, physical activity, or combinations thereof in order to regulate a patient's glucose with the goal of improving a patient's HbA1C value. For a high quality determination (i.e., coefficient of variation (CV) < 3%) of the HbA1C value, HbA1C assays are the norm in which blood samples are tested for the extent of glycation of hemoglobin by use of laboratory devices such as, for example, a D-10 analyzer from Bio-Rad Laboratories, or a G-7 analyzer from Tosoh Bioscience, Inc. For an approximated assessment of glycemia, HCPs alternatively use blood glucose (bG) values to determine an average glucose value, and then interpret the results to derive an estimated HbA1C value from spot monitoring blood glucose (SMBG) values. However, the estimated HbA1C value so obtained by such a prior art method, in general, is poor in quality (i.e., CV >5%).

Other prior art methods of solving a true mean bG value and estimating HbA1C value have been based on both the SMBG data collected during various clinical trials and the relationships derived therefrom. For example, many such prior art methods use SMBG data to develop prediction models based on statistical methods. Other methods consider weighted bG value schemas with additional predicators, such as a previous HbA1C value, to determine an estimated HbA1C value using a noted study relationship. While other methods further include transforming a bG value and then using the transformed bG value to determine the estimated HbA1C value. However, such prior art methods have the following potential issues: model parameters typically needs retuning, correlation is still generally poor (i.e., CV>5%), the standard errors are typically large, and adjustments to account for lifestyle related variations are not made such that any such reported patient specific solution is not specific enough to account for lifestyle related variations.

It is to be appreciated that one of the key limiting factors to finding a good generic algorithm which provides an accurate HbA1C prediction is the difficulty in obtaining comprehensive and detail (frequently sampled) blood glucose data under various conditions. For instance, studies having data sets based on continuous blood glucose monitoring, although providing dense data are typically conducted on relatively smaller population sizes and with durations that are relatively shorter in time than studies with SMBG data sets. With SMBG, on the other hand, there is a practical limitation of how many measurements can be collected. Since bG varies during the day, due to many factors such as physical activity, meal, and stress and so forth, it is not possible to get an accurate picture of a glucose excursion by just a few daily measurements. This means that the SMBG data sets (i.e., time-interval based data sets) often fail to capture true bG variation of the patient with diabetes (PwD). The implication is that the resulting prior art prediction models are normally then very study specific. Such prediction models therefore can neither be extended to account for other variables not addressed by the study(-ies) which they were based on nor used in an alternate situation to makes predictions without the need for an additional clinical trial to validate such model extensions. Finally, as such prior art methods fail to account for the context associated with bG measurements or in other words, to account for influence(s) of events such as carbohydrate ingestion, physical activity, insulin therapy, and so forth, such prior art methods are generally unsuitable for determining an estimated HbA1C value of good quality (i.e., CV <3%) for a patient specific lifestyle.

**Summary**

It is against the above background that the present invention addresses the above noted deficiencies in the prior art by disclosing a method, system, and computer program product for providing both an estimated true mean blood glucose value and estimated glycated hemoglobin (HbA1C) value of good quality (i.e., coefficient of variation preferably <3%) for a patient specific lifestyle from structured spot measurements of blood glucose.

In one embodiment, a computer-implemented method for providing both an estimated true mean blood glucose value and estimated glycated hemoglobin (HbA1C) value from spot blood glucose (bG) measurements is disclosed. The method comprises collecting both bG measurements and associated context of the bG measurement at daily times and events specified by a structured sampling schema; weighting each of the collected bG measurements based on the associated context; wherein the weighting is based on both duration between meals and age of bG measurement; determining the estimated true mean bG value and the estimate HbA1C value from the weighted measurements of the collected bG measurements; and providing the estimated true mean bG and the estimated HbA1C values.

In another embodiment, a system for providing both an estimated true mean blood glucose value and estimated glycated hemoglobin (HbA1C) value from spot blood glucose (bG) measurements is disclosed. The system comprises a display; memory; and a processor programmed: to collect both bG measurements and associated context of the bG measurement at daily times and events specified by a structured sampling schema provided in memory; to weight each of the collected bG measurements based on the associated context, wherein the weighting is based on both duration between meals and age of bG measurements; to determine the estimated true mean bG value and the estimated HbA1C value from the weighted measurements of the collected bG measurements; and to provide the estimated true mean bG and the estimated HbA1C values to the display.

In another example a system for providing both an estimated true mean blood glucose value and estimated glycated hemoglobin (HbA1C) value from spot blood glucose (bG) measurements is disclosed. The system comprises a blood glucose monitoring meter having memory and a first processor programmed to collect both bG measurements and associated context of the bG measurement at daily times and events specified by a structured sampling schema provided in the memory. The system further includes a computer having a display, memory and a second processor programmed: to receive the collected bG measurements and associated context from the meter; to weight each of the collected bG measurements based on the associated context; to determine the estimated true mean bG value and the estimated HbA1C value from the weighted measurements of the collected bG measurements; and to provide the estimated true mean bG and the estimated HbA1C values to the display.

In yet another example a computer program product is disclosed which comprises code that when executed by a processor based system performs the method steps of the present invention disclosed herein.

These and other advantages and features of the invention disclosed herein will be made more apparent from the description, drawings and claims that follow.

**Brief Description of the Drawings**

FIG. 1 depicts a tabulated dataset from simulation of a Mortensen Model based system with a simplification according to the present invention.

FIG. 2 depicts graphically HbA1C values generated by simulation which are plotted against mean bG values.

FIG. 3 depicts graphically a Gamma function profile.

FIG. 4 depicts graphically results obtained from simulation that show true mean bG is linearly related to HbA1C, whereby both positive and negative glycemic variations are illustrated.

FIG. 5 depicts graphically a normal daily lifestyle pattern of an individual for a modal day that consists of an overnight period and 3 meal types: breakfast, lunch and supper.

FIG. 6 depicts in block diagram meal selections from a glycemic excursion perspective categorized per meal type, meal amount, and meal speed.

FIG. 7 depicts graphically a grouping of glucose segments by event type to characterize and quantify the segments in order to help identify parameters that provided a high correlation ratio.

FIG. 8 depicts graphically 4 weighting schemes considered in developing a prediction model for HbA1C according to the present invention.

FIG. 9 depicts graphically a strong linear relationship between HbA1C and post prandial bG measurement when $t \geq 150$ minutes.

FIG. 10 depicts graphically quality of estimated HbA1C for various values of a Window Center (WinCen) and a Window Size (WinSize) for lifestyle context plotted by R-squared values.

FIG. 11 depicts graphically quality of estimated HbA1C for various values of a Window Center (WinCen) and a Window Size (WinSize) for lifestyle context plotted by mean squared errors.

FIG. 12 depicts graphically a comparison between daily lifestyle weighting and no daily lifestyle weighting and showing that daily life style weighting produces lower mean squared error.

FIG. 13 depicts graphically impact of visitation period (nDays) and number of sample (nSamples) for a WinCen of 190 minutes and a WinSize of 50 minutes.

FIG. 14 depicts graphically a sampling ratio plotted against R-squared values.

FIGS. 15A-E each depict graphically a sampling schema for sampled bG data being regressed and plotted by HbA1C %, and showing that the parameters for each linear regression are in close proximity to each other.

FIGS. 16A-E each depict graphically a sampling schema for sampled bG data being regressed and plotted by HbA1C % with a prediction line (center line) and a 95% confidence interval (CI) boundaries (above and below curves) shown in the subplots.

FIG. 17 depicts in block diagram a processor based system for implementation of the present invention.

FIG. 18 is a flow diagram of one embodiment of a method for implementation of the present invention.

FIG. 19 is a flow diagram of another embodiment of a method for implementation of the present invention.

## Detailed Description

It is to be appreciated that embodiments of the present invention enhance existing software and/or hardware that retrieves and processes blood glucose (bG) data. The embodiments of the invention can be directly incorporated into existing home glucose monitors, or used for the enhancement of software that retrieves and processes bG data, by introducing a method for providing an estimated true mean blood glucose value and estimated glycated hemoglobin (HbA1C) value of good quality from structured spot measurements of blood glucose having a coefficient of variation (CV) of less than 5% in one embodiment, and less than 3% in a preferably embodiment. The invention further relates to a computer program for implementing the method for providing both the estimated true mean blood glucose value and the estimated glycated hemoglobin (HbA1C) value from the structured spot measurements of blood glucose and life style information.

In the sections to follow, a discussion is made first to the approach used to derive the equations for providing the estimated true mean blood glucose (bG) value and the estimated glycated hemoglobin (HbA1C) value from structured spot measurements of blood glucose (i.e., bG data) collected, as per a measurement schema according to the present invention. It is to be appreciated that the measurement schema according to the present invention assumes that the PwD maintains a repeatable average behavior, whereby collection exceptions (i.e., missed testing times) are managed by the algorithm on the estimated HbA1C value. It is further to be appreciated that the utility of providing an estimated HbA1C value that demonstrates continuous blood glucose monitoring will provide a fairly accurate idea of the overall level of glycemia of the PwD, as compared to the uncertainty associated with estimating glycemia and its variation based only on spot monitoring. Additionally, certain HbA1C values have been linked to various disease states, and thus having a good estimated HbA1c value between laboratory based assays values can help identify much earlier a patient's potential risk associated to long term complications, such as micro-vascular disease complications. Furthermore, providing an assessment of overall glycemia via an estimated HbA1C value of good quality can empower the PwD to better manage his/her diabetes. A discussion of the methodology used to provide the estimated true mean blood glucose value and estimated glycated hemoglobin (HbA1C) value from structured spot measurements of blood glucose (i.e., bG data) collected, as per a measurement schema according to the present invention, now follows.

*Kinetics of glycation of hemoglobin*

Glycation is a non-enzymatic chemical reaction wherein the glucose molecules bind with the amino acid groups of the proteins. Of the many glycated proteins hemoglobin A1C is fairly stable and one of the dominant forms of glycohemoglobin. Synthesis of HbA1C is primarily a condensation of hexose with the hemoglobin structure to form an unstable intermediate Schiff base adduct, or aldimine, followed by the Amadori rearrangement to form the stable ketoamine adduct, HbA1c. The kinetics of the glycation of hemoglobin to surrounding glucose concentration can be modeled by three differential equation, Equations (1)-(3), which are disclosed more fully by the publication of Mortensen, H. B.; "Glycated hemoglobin. Reaction and biokinetic studies. Clinical application of hemoglobin A1c in the assessment of metabolic control in children with diabetes mellitus," Danish medical bulletin (1985), 32(6), pp. 309-328. The model according to Equations (1)-(3), is referred herein as the Mortensen Model.

*Mortensen Model*

$$\frac{dH_{bA}}{dt} = -k_{12}H_{bA}G + k_{21}H_{bA1d} \tag{1},$$

$$\frac{dH_{bA1d}}{dt} = k_{12}H_{bA}G - \left(k_{21} + k_{23}\right)H_{bA1d} + k_{32}H_{bA1C} \tag{2},$$

and

$$\frac{dH_{bA1c}}{dt} = k_{23}H_{bA1d} - k_{32}H_{bA1C} \qquad (3).$$

In the Mortensen model, the term $H_{bA}$ represents the sub-pool of erythrocytes of same age, whereby the pool consists of cohorts of erythrocytes of varying age. The behavior of each cohort is represented by a corresponding set of Equations (1)-(3). From the Mortensen publication, the $k$ parameters used in the model are known as follows: $k_{12}$ = 5.76 mmol/l/min; $k_{21}$ = 0.006 /min; $k_{23}$ = 0.000852 /min; and $k_{32}$ = 0.000102 /min. Next, to test the utility of the Mortensen model in helping to generate a basic relation between HbA1C and bG, glycation simulations were run on simulated data for meal related bG excursions which is discussed hereafter.

*Glycation simulation setup*

Meal related bG excursions were evaluated first using simple mathematical formulas, whereby simulated data helped to generate a basic relation between HbA1C and bG. It is to be appreciated that the glycation of erythrocytes is a continuous process. However, the erythrocytes have finite lifespan of approximately 120 days. Depending on problem needs one can use other lifespan values such as ranging more or less from 90 ~ 120 days to cover different population groups and/or physiological conditions. This means that in addition to the glycation, erythrocytes are continuously being added and removed from the glycation process. As the aged erythrocytes are replaced, the state of glycation of all the cells has to be managed. From a simulation perspective, instead of using Equations (1)-(3) for each cell, a simplification was done by grouping cells into cohorts of equally aged cells. In particular for the glycation simulation setup, $n$ numbers of cohorts of erythrocytes were considered, whereby each of the cohorts was described by the set of the 3 differential equations (Equations (1)-(3)). Each cohort is assumed to have a life span of $n$ days. When a cohort's maximum age is reached a new cohort replaces it. The simulation handled this by resetting the 3-states of the oldest cohort (i.e. when the age of the cohort reaches its life-duration of 120 days) to the state of a fresh cohort of erythrocytes with un-glycated hemoglobin. In all, there were $n$ sets of differential equations used in the simulation, whereby each set of equations represented a state of the corresponding cohort.

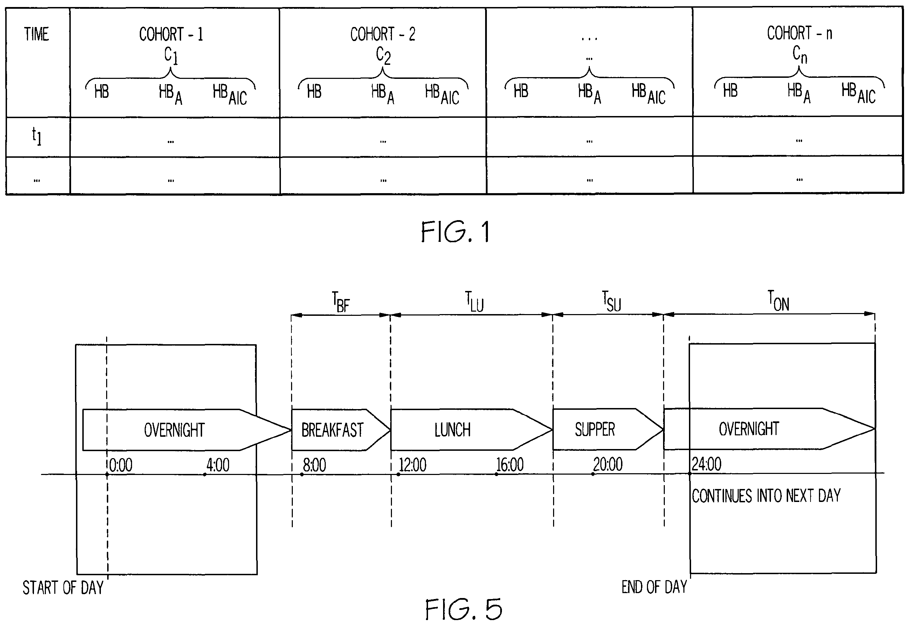

The 3n states were stored in columns as shown schematically in FIG. 1, which represent a tabulated dataset from the simulation of the Mortensen Model system using the above mentioned simplification. At each new time instant, the values for each of the states were recorded in the next new row as a record set, whereby glycation of each cohort at any given time is the value of the 3$^{rd}$ state. The net HbA1C in percentage (%) can be given by summing the HbA1C state for each of the cohort according to Equation (4):

$$HB_{A1C} = 100 \sum_{i=3,9,..}^{n} x_i \qquad (4),$$

where the summation counter i is the column corresponding to the $Hb_{A1C}$ state. Using Equations (1)-(4), HbA1C can be simulated for an arbitrary bG profile. The true mean blood glucose value, $\overline{bG}$ can be thus given by Equation 5:

$$\overline{bG} = \frac{AUC}{Duration} \qquad (5),$$

where AUC is the area under a continuous bG excursion curve. However, when bG measurements are sparse and non-continuous, as in the case of spot bG measurements, then it is to be appreciated that Equation 5 is no longer valid. Accordingly, a new relationship was derived in order to estimate the true mean bG as follows.

*Simulated Cases*

Under the above mentioned idealized setup, the relationship between periodic glucose profiles and corresponding HbA1C values was then examined for deriving useful insights and relationships. Specifically, two profiles were examined: (1) Sinusoidal glucose profile with offset (Equation (6)); and (2) Gamma function profile with offset (Equation (8)). These functions can be seen as representative of the meal event with post-prandial glucose behavior for varying levels of control, whereby constant glucose is a special case of both of the functions. For the sinusoidal glucose profile, Equation (6) is defined as:

$$bG = bG_{const} + \frac{A}{2}\left(1 - \cos\left(\frac{2\pi}{T}t\right)\right) \qquad (6),$$

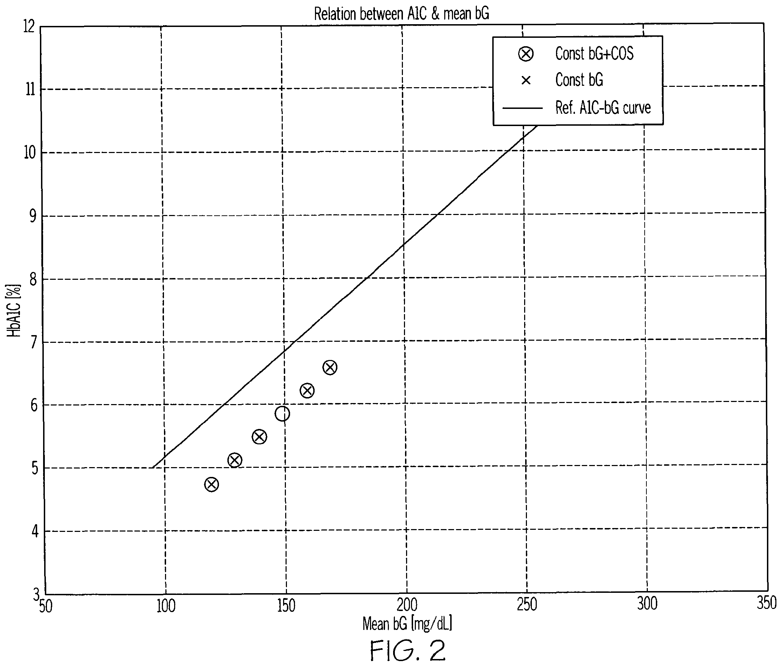

where, $bG_{const}$ provides the steady state offset and $\frac{A}{2}\left(1 - \cos\left(\frac{2\pi}{T}t\right)\right)$ is the cosine curve with amplitude A and period T. FIG. 2 shows the HbA1C values generated by the simulation plotted against the mean bG values. The results in FIG. 2 show that the true mean bG for the continuous glucose profile and simulated HbA1C is approximately linear as shown by the 'o' symbols. It also shows that HbA1C obtained by a constant bG and that from oscillating bG are approximately identical if they have the same mean bG value. If comparing the HbA1C resulting from two sinusoidal inputs, which are identical except for the frequency, the HbA1C from the slower varying signal will have comparatively higher glycation rate. The rate of signal has an effect but under conditions of interest it is small. The solid curve shows a known relationship between HbA1C and mean bG and is used as a reference.

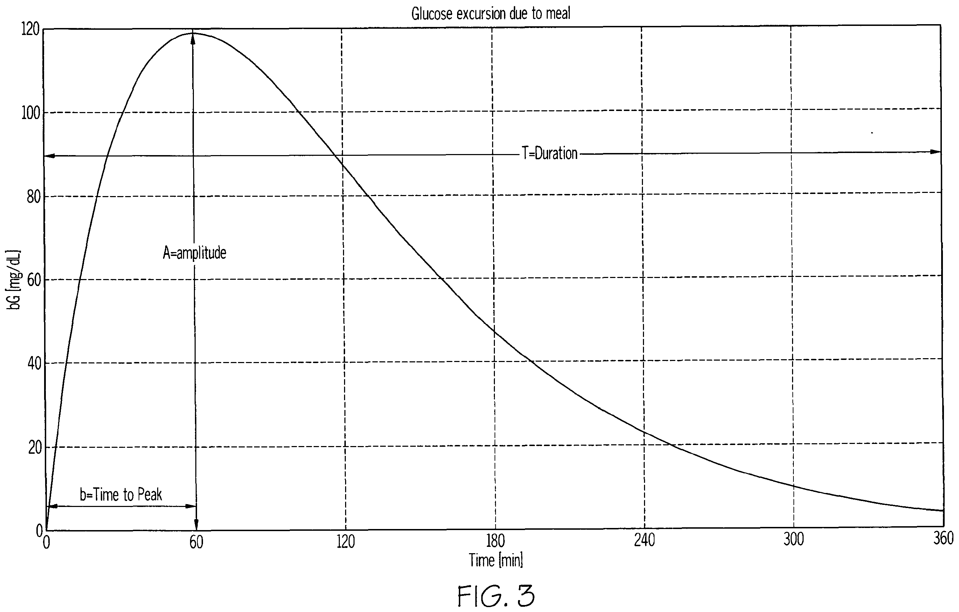

The Gamma function profile is shown by FIG. 3, and is described by the function defined by Equation (7):

$$f(t) = \frac{t^{\alpha-1}e^{-1/\beta}}{\beta^{a}\Gamma(\alpha)}, t \geq 0 \qquad (7).$$

In this simulation embodiment, the Gamma function was used to represent the post meal glucose excursion. Grossly approximated, the model shows a two (2) compartment model of glucose in a post prandial state. It has been used primarily to understand the impact of glucose varying from the aspects of different rates of post prandial rise, decay and magnitude of an excursion. The parameters $\alpha$ and $\beta$ approximately represent the number of compartments and time to peak. In fact, if $\alpha$ is set to 2, a 2nd order compartment model is considered with a time constant for both compartments equal to $\beta$ (2nd order system with repeated poles). Therefore, the above function according to Equation (7) simplifies to:

$$f(t) = \frac{te^{-1/\beta}}{\beta^{2}},$$ $t \geq 0$. The peak value of the function $f(t)$ is reached when t=$\beta$. The peak value is then $\frac{1}{e\beta}$. Therefore, the glucose excursion used herein can be defined by Equation 8:

$$bG(t) = bG_{const} + \frac{Ae}{\beta}te^{-1/\beta}, t \geq 0 \qquad (8),$$

where $A$ is the peak bG value with respect to $bG_{const}$.

The response of HbA1C to glucose excursions for various time to peak and peak values was then studied both analytically and in simulation. The Gamma functions for the various combinations of the parameters studied are listed in Table 1.

**Table 1: Parameter settings for Gamma function**

| Parameter | Values |
|---|---|
| Constant, $bG_{const}$ [mg/dL] | 80, 100, 120, 140 |
| Amplitude, A [mg/dL] | Positive (+ve) glucose push<br>0, 40, 60, 80, 100, 120, 140, 180, 300 |
| | Negative (-ve) glucose push<br>0, 40, 60, 80 |
| Periodicity [hour] | 4, 6, 8, 12, 24 |
| Time to peak, $\beta$ [minute] | 30, 60, 90, 120 |

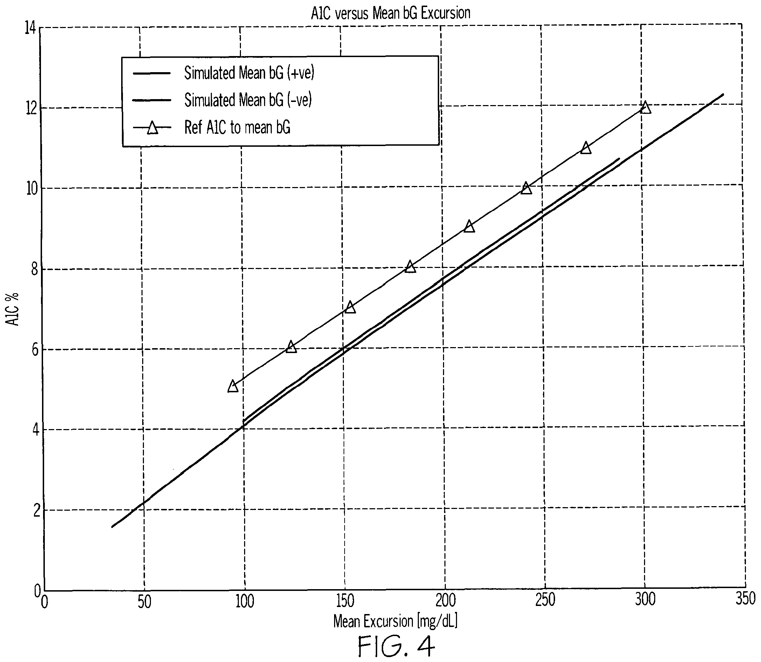

The results obtained from simulation show that the true mean bG is linearly related to HbA1C. This linear relationship is shown by FIG. 4, whereby both positive and negative glycemic variations are illustrated. The solid line above 4% HbA1C is for positive glycemic variation with respect to a constant 100 mg/dL signal and the dashed line below 4% HbA1C is for the negative glycemic variation. In order to provide a more accurate (and thus more useful) estimated HbA1C value e.g., having less than 3% CV, a more accurate estimated true mean bG is needed. However, it is to be appreciated that in the case of spot measurement devices, increasing the data set of bG measurements upon which to base a more accurate estimated true mean bG is not practical as a PwD can only tolerably comply with about 3 to 6 measurements daily for normal day-to-day use. This is not to say that such a data set of bG measurements cannot be increased such as, for example, for an occasional, short term (i.e., 1 to about 7 days) testing protocol in which the required daily spot measurements are more than what the PwD would be expected to tolerably comply with for normal day-today use (e.g., greater than 6 measurements daily). Additionally, bG values are used in intensive insulin therapy to primarily regulate glucose to target. This means bG measurement timings are dependent on the requirements of intensive therapy and not on providing a better estimate of true mean bG. This is especially true in the case of Type I diabetic patients. Furthermore, for practical considerations the bG measurement cannot be limited to a specific time instant. And finally, the data analyzed normally covers two consecutive patient visits to the HCP. The period between visitation can range from 3 to 4 months. Accordingly, with the above issues in mind, specifying a time window for bG measurement was determined by the inventors to be more realistic. These issues were examined analytically using a gamma function profile, which is discussed hereafter in later sections. An example of a normal lifestyle of a PwD is now provided in order to illustrate the lifestyle aspects and context-based measurements that are collected according to a measurement schema of the present invention.

*Lifestyle aspects and Context based measurements*

Intensive therapy addresses the occurrences of bG excursion and provides insulin dosing rules for correcting events such as meals, exercise, etc. This leads to the term "lifestyle" which captures the properties/characteristics of the occurrences of meal events, exercise events, etc., for a PwD. Lifestyle thus has a strong connotation of daily habits. In the following example, the habits are limited to meals, but other embodiments may be extended to include other events captured, for example, physical activity, intake of oral drugs, and other daily activities.

In the following example, one daily lifestyle pattern (habit) examined consisted of an overnight period and a day period consisting of multiple meals and snacks for a patient. The daily lifestyle pattern repeats itself over a period of months whereby the timing of meals varied randomly around expected meal times. The size and composition of the meal was similarly modeled by assigning the parameters of the gamma function values generated from statistical distribution. In general, it was assumed that by considering more or less a 3 month time frame, the persistent average behavior would be observed in HbA1C value even though from meal to meal there could be potentially large variability. Thus, in the given example, a modal day consisted of an overnight period and 3 meal types: breakfast, lunch and supper as is shown in FIG. 5, which is an example of a normal lifestyle for an individual. It is to be appreciated that snacks are ignored in this illustrated embodiment for simplicity but such can be introduced in other embodiments without impacting the general approach. From either questionnaire or systematic data collection, time periods covering these events are collected.

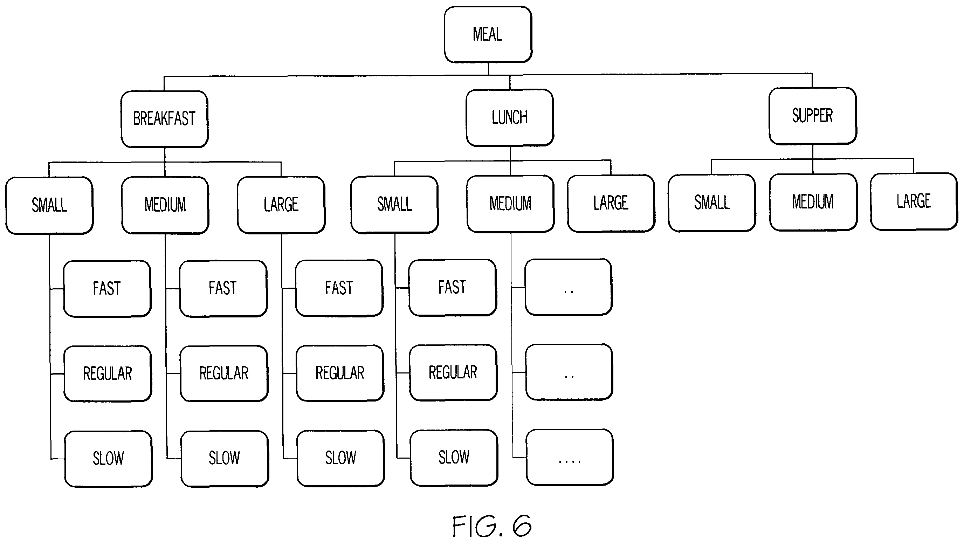

Further complexity in glucose excursion characteristics is addressed by modeling a range of meal content characterized generally by amount and speed. Meal content is given by meal composition and amount of meal which relates to speed and duration of glucose absorption. It is observed that individuals have repeatability in their meal selection, which from a glycemic excursion perspective can be classified by its speed and amount. In one embodiment, as shown in FIG. 6, meals are categorized per meal type, meal amount, and meal speed. Similarly, in other embodiments, additional meals (or less meals if such more accurately reflects a patient's eating habits), exercise (physical activity), stress, alternate states, and medication can be characterized and modeled. For example, an alternate state category may be used to capture the change in physiological metabolic state, such as brought on by stress, a menstrual cycle, or exercise, which leads to change in insulin resistance, insulin sensitivity, glucose utilization, and so forth.

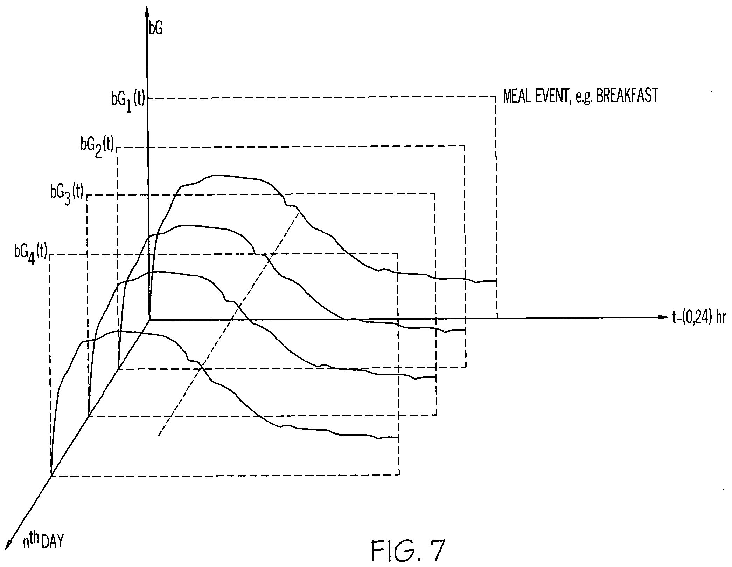

Mathematically, then, statistical properties were assigned to each of the categories. As per the above description, meals were further classified by 3 broad categories of meal speed: fast, regular and slow, and meal amount is similarly classified into 3 categories as: small, medium and large. Other terms used were less than normal, normal and more than normal. The latter part described better the majority of the cases. For purposes of simplification of the simulation, physical activity was assumed to be fixed. Thus, grouping glucose segments by event type, for instance meals, and further sub-grouping them by characterizing the meal size and speed, allows one to characterize and quantify them, which is illustrated by FIG. 7. Such context based grouping and then examining the average behavior helped to identify parameters that provided a high correlation ratio. Using the normal lifestyle described above, along with the nine meal categories, a fairly wide range of post prandial behavior for an individual is covered. The gamma function according to Equation 8 was then used in the analytical analysis as well as in simulation to study the impact of lifestyle in the derivation of the relation between HbA1C and bG measurements. It is to be appreciated that how the lifestyle pattern is segmented and correlated can be varied based upon each patient observed habits and by using other distribution methods in other embodiments.

It is to be appreciated that in reality the glucose profiles of a PwD are richer in their response and are potentially harder to characterize. The richness is associated with multiple physiological factors influencing the overall glucose state. However, assuming that the meal related glucose push is dominant, the PwD is working towards regulating glucose to a target value by means of medications, diet control and exercise. Inherently there is an objective of achieving euglycemia at all times. By averaging many such responses, however, the glucose effect on glycemia can be estimated based on the relations derived using the gamma function. The arbitrary meal response curves shown in FIG. 7 are thus represented by the gamma function (Equation 8), which was then used to derive a relation for true mean bG and peak amplitude A. Additionally, the following provides a theoretical basis for the new lifestyle based approach.

*Mean value for Gamma Function*

It is to be appreciated that the gamma function $f(t)$ is neither symmetric nor periodic. We define parameter T which is the time duration between consecutive meal. Considering the exponential properties, the decay of a pure exponential curve to 99% of its starting value is equal to 4 times the time constant. Therefore, the gamma function according to Equation 8 is basically a $2^{nd}$ order differential equation with repeated poles. The time constant for the gamma function is thus $\dfrac{1}{\beta}$, and the mean value can be determined by considering the waning factor $n$, which is defined as: $n = \dfrac{T}{\beta}$ or $T = n*\beta$, where $n = 3, 4$.

The mean bG value for $\dfrac{Ae}{\beta} t e^{-t/\beta}$ (from Equation 8) is now derived. If we consider, $g(t) = \dfrac{Ae}{\beta} \dfrac{1}{T} \int_0^T t e^{-t/\beta} dt$, and integrate $g(t)$ by parts, the following Equations (9)-(13) are provided:

$$g(t) = \frac{Ae}{\beta} \left( \frac{1}{T} \left[ t \frac{e^{-t/\beta}}{-1/\beta} \right]_0^T - \frac{1}{T} \int_0^T (1) \frac{e^{-t/\beta}}{-1/\beta} dt \right), \qquad (9)$$

$$g(t) = \frac{Ae}{\beta} \left( \frac{1}{T} \left[ t \frac{e^{-t/\beta}}{-1/\beta} \right]_0^T - \frac{1}{T} \left[ \frac{e^{-t/\beta}}{(-1/\beta)(-1/\beta)} \right]_0^T \right), \qquad (10)$$

$$g(t) = \frac{Ae}{\beta} \left( \frac{1}{T} \left[ t \frac{e^{-t/\beta}}{-1/\beta} \right]_0^T - \frac{1}{T} \left[ \beta^2 e^{-t/\beta} \right]_0^T \right), \qquad (11)$$

$$g(t) = \frac{Ae}{\beta} \left( -\frac{1}{T} \left[ t\beta e^{-t/\beta} + \beta^2 e^{-t/\beta} \right]_0^T \right), \qquad (12)$$

$$g(t) = \frac{Ae}{\beta} \left( -\frac{1}{n\beta} \left[ n\beta\beta e^{-n\beta/\beta} + \beta^2 e^{-n\beta/\beta} \right] + \frac{1}{n\beta} \left[ 0 + \beta^2 e^{-0/\beta} \right] \right), \quad (13)$$

where $T = n\beta$. Note, however, the mean value is a function of $\beta$, but if $T$ is expressed in terms of $\beta$, then $\beta$ falls out, which further simplifies to $g(t) = \frac{Ae}{\beta} \left( \frac{1}{n} \beta \left( 1 - (n+1)e^{-n} \right) \right)$, and finally, $\overline{bG} = \frac{Ae}{n} \left( 1 - (n+1)e^{-n} \right)$. In this manner, when the waning factor $n$ equals 3, the mean value $\overline{bG}$ is 0.726$A$, and when the waning factor $n$ equals 4, the mean value $\overline{bG}$ is 0.617A. Thus, the mean value $\overline{bG}$ is a function of amplitude $A$ and the waning factor $n$. Adding the basal glucose level term $bG_{Const}$, the mean value $\overline{bG}$ can be then defined by Equation (14) as:

$$\overline{bG} = \frac{Ae}{n}\left(1-(n+1)e^{-n}\right)+bG_{Const} \qquad (14).$$

Thus, if a peak bG value is measured, the mean value $\overline{bG}$ could be estimated since the waning factor $n$ given

$$n = \frac{DurationBetweenMeal, T}{TimeToPeak, \beta}.$$

the lifestyle can be determined by So, for a given gamma function one could simply state that for the mean value, $\overline{bG} = kA + bG_{Const}$. Based on simulation of the Mortensen model, as shown by FIG. 2, it is noted that HbA1C is linearly related to true mean bG. Thus, HbAC1 may be defined by Equation (15) as:

$$HbA1C = K\overline{bG} + \text{constant} \qquad (15).$$

From the above derivation it is also clear that both meal size and meal duration (associated with speed) influences the degree of glycation. Next, a discussion of the process used to characterize a PwD's lifestyle is provided. Equation (15) is central to derivations presented in latter paragraphs.

*Lifestyle (meal only)*

As discussed above, the day, as per the lifestyle, is divided into appropriate segments where the bG traces for each day are segmented and each like segments grouped (e.g., FIG. 7). The bG data covering number of days are grouped into segments as mentioned in the above embodiment comprise: a fasting segment, a breakfast segment, a lunch segment, and a supper segment. Starting from continuously sampled data, the mean value $\overline{bG}$ is approximately given by Equation (16) as:

$$\overline{bG} = \frac{\sum_{i=1}^{n} bG_i}{n} \qquad (16).$$

For a meal related segment, the gamma function is described by the peak value A with respect to the basal or fasting bG and time to peak, $\beta$ for bG. The parameters are summarized in Table 2.

**Table 2: Meal characteristics**

|  | **Fast** | **Regular** | **Slow** |
|---|---|---|---|
| **Small** | $A_S^{BF}$ , $\beta_{Fast}^{BF}$ | $A_S^{LU}$ , $\beta_{Regular}^{LU}$ | $A_S^{SU}$ , $\beta_{Slow}^{SU}$ |
| **Medium** | $A_M^{BF}$ , $\beta_{Fast}^{BF}$ | $A_M^{LU}$ , $\beta_{Regular}^{LU}$ | $A_M^{SU}$ , $\beta_{Slow}^{SU}$ |
| **Large** | $A_L^{BF}$ , $\beta_{Fast}^{BF}$ | $A_L^{LU}$ , $\beta_{Regular}^{LU}$ | $A_L^{SU}$ , $\beta_{Slow}^{SU}$ |

In terms of analysis then, the meals are then characterized to cover a time period, such as for example, a 2-4 month period between HCP visitations, in the following manner. For breakfast type meals, the total number of breakfasts is represented by the term $m^{BF}$, and the ratio of the number of small breakfast meals, medium breakfast meals, large breakfast meals and no breakfast meals are represented by $\alpha_{SMALL}^{BF}$, $\alpha_{MED}^{BF}$, $\alpha_{LARGE}^{BF}$ and $\alpha_{\phi}^{BF}$, respectively. Total breakfasts $m^{BF}$ can then be defined according to Equation (17) as:

$$\alpha_{SMALL}^{BF} m^{BF} + \alpha_{MED}^{BF} m^{BF} + \alpha_{LARGE}^{BF} m^{BF} + \alpha_{\phi}^{BF} m^{BF} = m^{BF} \qquad (17).$$

Similarly, meal speeds for fast, regular, and slow meals are represented by the terms: $\lambda_{FAST}^{BF}$, $\lambda_{REG}^{BF}$, and $\lambda_{SLOW}^{BF}$, respectively. Therefore, total breakfasts $m^{BF}$ can also be defined according to Equation (18) as:

$$\lambda_{FAST}^{BF} m^{BF} + \lambda_{REG}^{BF} m^{BF} + \lambda_{SLOW}^{BF} m^{BF} + \alpha_{\phi}^{BF} m^{BF} = m^{BF} \qquad (18).$$

It is assumed that on average for each meal amount category there is a breakdown for meal speed with the same ratios. In other words, for example, small breakfast meals $m_{SMALL}^{BF}$ can be defined according to Equation (19) as:

$$\lambda_{FAST}^{BF} \alpha_{SMALL}^{BF} m^{BF} + \lambda_{REG}^{BF} \alpha_{SMALL}^{BF} m^{BF} + \lambda_{SLOW}^{BF} \alpha_{SMALL}^{BF} m^{BF} = m_{SMALL}^{BF} \qquad (19).$$

Equation (16) the $bG_i$ terms on the right hand side are grouped as per FIG. 5 to derive a simplified mean glucose relationship using relationship $\overline{bG} = kA + bG_{Const}$ for a Gamma function of amplitude $A$ (derived earlier). FIG. 5 in this example consists of overnight and 3 meal segments breakfast, lunch and supper. The overnight part of the day in this example is generally the sleep period. During this period the physical activity is minimal. Meal affects are waning out, insulin bolus affects are also petering out. There are other effects such as, for example, the dawn phenomenon caused by growth hormones, which are especially dominant in adolescents. However, it is anticipated that during the overnight period, the overnight mean blood glucose value, represented by the term $\overline{bG}_{ON}$, is converging to a desired target. Accordingly, $\overline{bG}_{ON}$ is the mean bG obtained by considering all bG values covering a fasting segment, and covering all the overnight segments. The mean bG component for the overnight segment is then given by Equation (20) as:

$$\overline{bG_1} = \frac{T_{ON}}{24} \overline{bG}_{ON} \qquad (20),$$

where $T_{ON}$ covers time duration for overnight part as illustrated in FIG 5.

What can constitute fasting bG values requires more specifics. For example, pre-meal bG measurements could be grouped as fasting bG values under certain conditions, overnight bG measurements, early morning bG measurements. Average of such measurements approximately represents the mean bG for the overnight period. Then the component required for $\overline{bG}_{ON}$ is given by Equation (21) as:

$$\overline{bG}_{ON} = \overline{bG}_{Fasting} \qquad (21).$$

Next, given the first predictor term $\overline{bG}_{Fasting}$, which covers the overnight period, the remaining are the meals related excursion with respect to $\overline{bG}_{Fasting}$. So each of the meal which are gamma function thus can be defined according to Equation (22) as:

$$\overline{bG} = KA + \overline{bG}_{FASTING} \qquad (22),$$

where $A$ is the peak disturbance with respect to $\overline{bG}_{Fasting}$.

Determination of "A " for the case when various glucose excursions due to different meals is now explained. As explained earlier and summarized by FIG. 5 and FIG. 6 the excursions are due to the 3 normally eaten meals and then each meal characterized by its size and speed. For illustration purpose, consider the breakfast part first. Next, if consider small breakfast meals and include all meal speeds, then the area under the gamma function according to Equation (23) as:

$$\left(\alpha_{SMALL}^{BF}m^{BF}\right)T^{BF}\overline{bG}_{SM}^{BF}-\overline{bG}_{FASTING}=T^{BF}\sum_{i=1}^{\alpha_{SMALL}^{BF}m^{BF}}K_i^{BF}A_{SMALL,i}^{BF} \quad (23),$$

which covers all small breakfast meals. The term $T^{BF}$ is the time duration between start of breakfast to start of lunch. Similar equations can be written for medium and large breakfasts, which when combined results in Equation (24), which is defined as:

$$m^{BF}T^{BF}\overline{bG}-\overline{bG}_{FASTING}=\underbrace{T^{BF}\sum_{i=1}^{\alpha_{SMALL}^{BF}m^{BF}}K_i^{BF}A_{SMALL,i}^{BF}}_{Term-1}+\underbrace{T^{BF}\sum_{i=1}^{\alpha_{MED}^{BF}m^{BF}}K_i^{BF}A_{MED,i}^{BF}}_{Term-2}+\dots$$

$$\underbrace{T^{BF}\sum_{i=1}^{\alpha_{LARGE}^{BF}m^{BF}}K_i^{BF}A_{LARGE,i}^{BF}}_{Term-3}+\underbrace{T^{BF}\sum_{i=1}^{\alpha_{\phi}^{BF}m^{BF}}K^{BF}A_{\phi}^{BF}}_{Term-4}$$

$$(24).$$

The term $A_{\phi}^{BF}$ is of course zero. The number of meals considered in the equation covers a time window of interest. Such a window may range from 2 months to 4 months, or may be as few as 3 day to 30 days, if an estimated prediction is desired as explained in a later section.

If Term-1 is considered, then the term $K_i^{BF}$, meal speed, can now be factored out as a constant. The result is shown by Equation (25).

$$\sum_{i=1}^{\alpha_{SMALL}^{BF}m^{BF}}K_i^{BF}A_{SMALL,i}^{BF}=K_{FAST}^{BF}\sum_{i=1}^{\lambda_{FAST}^{BF}\alpha_{SMALL}^{BF}m^{BF}}A_{SMALL,i}^{BF}+K_{REG}^{BF}\sum_{i=1}^{\lambda_{REG}^{BF}\alpha_{SMALL}^{BF}m^{BF}}A_{SMALL,i}^{BF}+K_{SLOW}^{BF}\sum_{i=1}^{\lambda_{SLOW}^{BF}\alpha_{SMALL}^{BF}m^{BF}}A_{SMALL,i}^{BF} \quad (25).$$

It is to be appreciated that the PwD categorizes and provides the size of meals as small, medium large meal amounts, as well as the meal speed. For instance, all small meals can be simply represented by an average value $\overline{A}_{SMALL}^{BF}$. Thus, for example, all fast small meals may be represented by Equation (26) as:

$$\lambda_{FAST}^{BF}\alpha_{SMALL}^{BF}m^{BF}\overline{A}_{SMALL}^{BF} \quad (26).$$

Collecting all the terms together, Equation (25) then can be rewritten as Equation (27) as:

$$\sum_{i=1}^{\alpha_{SMALL}^{BF}m^{BF}}K^{BF}A_{SMALL}^{BF}=\alpha_{SMALL}^{BF}m^{BF}\left(\lambda_{FAST}^{BF}K_{FAST}^{BF}+\lambda_{REG}^{BF}K_{REG}^{BF}+\lambda_{SLOW}^{BF}K_{SLOW}^{BF}\right)\overline{A}_{SMALL}^{BF} \quad (27).$$

Now considering all the meal types we get the following relation shown by Equation (28) is follows:

$$m^{BF} T^{BF} \overline{bG} - \overline{bG}_{FASTING} = \underbrace{T^{BF} \alpha_{SMALL}^{BF} m^{BF} \left( \lambda_{FAST}^{BF} K_{FAST}^{BF} + \lambda_{REG}^{BF} K_{REG}^{BF} + \lambda_{SLOW}^{BF} K_{SLOW}^{BF} \right) \overline{A}_{SMALL}^{BF}}_{Term-1} +$$

$$\underbrace{T^{BF} \alpha_{MED}^{BF} m^{BF} \left( \lambda_{FAST}^{BF} K_{FAST}^{BF} + \lambda_{REG}^{BF} K_{REG}^{BF} + \lambda_{SLOW}^{BF} K_{SLOW}^{BF} \right) \overline{A}_{MED}^{BF}}_{Term-2} +$$

$$\underbrace{T^{BF} \alpha_{LARGE}^{BF} m^{BF} \left( \lambda_{FAST}^{BF} K_{FAST}^{BF} + \lambda_{REG}^{BF} K_{REG}^{BF} + \lambda_{SLOW}^{BF} K_{SLOW}^{BF} \right) \overline{A}_{LARGE}^{BF}}_{Term-3} +$$

$$\underbrace{T^{BF} \sum_{i=1}^{\alpha_\phi^{BF} m^{BF}} K^{BF} A_\phi^{BF}}_{Term-4}$$

$$(28).$$

On further simplification, Equation (28) becomes:

$$\overline{bG}^{BF} - \overline{bG}_{FASTING} = \left( \lambda_{FAST}^{BF} K_{FAST}^{BF} + \lambda_{REG}^{BF} K_{REG}^{BF} + \lambda_{SLOW}^{BF} K_{SLOW}^{BF} \right) \left( \alpha_{SMALL}^{BF} \overline{A}_{SMALL}^{BF} + \alpha_{MED}^{BF} \overline{A}_{MED}^{BF} + \alpha_{LARGE}^{BF} \overline{A}_{LARGE}^{BF} \right)$$

The last group of terms on the right-hand side are the weighted amplitude terms which is the average amplitude. Thus, equation (28) can be further rewritten as: $\overline{bG}^{BF} - \overline{bG}_{FASTING} = \left( \lambda_{FAST}^{BF} K_{FAST}^{BF} + \lambda_{REG}^{BF} K_{REG}^{BF} + \lambda_{SLOW}^{BF} K_{SLOW}^{BF} \right) \overline{A}^{BF}$ .

And $\left( \lambda_{FAST}^{BF} K_{FAST}^{BF} + \lambda_{REG}^{BF} K_{REG}^{BF} + \lambda_{SLOW}^{BF} K_{SLOW}^{BF} \right)$ is a factor for given lifestyle characteristics. Similarly for other meals, relations can be derived, such as: $\overline{bG}^{LU} - \overline{bG}_{FASTING} = \left( \lambda_{FAST}^{LU} K_{FAST}^{LU} + \lambda_{REG}^{LU} K_{REG}^{LU} + \lambda_{SLOW}^{LU} K_{SLOW}^{LU} \right) \overline{A}^{LU}$ , and

$\overline{bG}^{SU} - \overline{bG}_{FASTING} = \left( \lambda_{FAST}^{SU} K_{FAST}^{SU} + \lambda_{REG}^{SU} K_{REG}^{SU} + \lambda_{SLOW}^{SU} K_{SLOW}^{SU} \right) \overline{A}^{SU}$ . So the final mean value of bG for a modal day can be defined according to Equation (29) as:

$$\overline{bG} = \frac{T^{FASTING}}{24} \overline{bG}_{FASTING} + \frac{T^{BF}}{24} \overline{bG}^{BF} + \frac{T^{LU}}{24} \overline{bG}^{LU} + \frac{T^{SU}}{24} \overline{bG}^{SU} \qquad (29).$$

The mean values $\overline{bG}^{BF}$, $\overline{bG}^{LU}$ and $\overline{bG}^{SU}$ represents mean bG for corresponding meal segment. The above result Equation (29) shows that the specifics of the meal in the final meal equation collapse into a simple average relation in which the averages of an individual event is time weighted as is shown by Equation (29). The above conclusion according to the present invention was verified in simulation (FIG. 4). Relation 29 forms the basis to segment the day as per lifestyle event and examine it from the perspective of replacing it by a meaningful average value. Another fundamental aspect to the algorithm is temporal weighting schema. The affect of past breakfast on current HbA1C is not equally weighted. Such weight schema is theoretically derived based on the assumption of lifespan of the erythrocytes.

*Temporal Weighting*

Temporal weighting of bG values becomes relevant when the prediction model is derived between SMBG values and HbA1C. As mentioned previously above, each cohort has a finite life span of approximately 120 days. Thus, for this example, a lifespan of 120 days is considered. The aged cells are constantly being replaced by young erythrocytes. So at any given time each of the cohort's age will range from 0 to 119 days. Each cohort thus is exposed to a subset of corresponding bG data. Considering the glycated hemoglobin at current time and all the bG values over the last 120 days, then the bG value that is 120 days old influences only 1 out of 120 cohorts and none of the other cohorts with ages less than 120 days. On the other hand, the current bG value affects all ages of the surviving cohorts i.e. the last 120 cohorts. In context of constant bG and considering the physiological aspect, this suggests that an appropriate weighted mean bG value can help improve HbA1C prediction.

In a simulation exercise, a lifespan L was set to 120 days and a number of cohorts N was made equal to 120 cohorts, where cohort # 120 is the oldest cohort, and cohort #1 is the newest. For a cohort aged L days (considering the oldest cohort), then the impact of $bG_i$ on HbA1C can be approximated according to Equation (30) as:

$$\frac{\sum_{i=1}^{L} bG_i \Delta T}{\sum_{i=1}^{L} \Delta T} = \frac{1}{L} \sum_{i=1}^{L} bG_i \qquad (30),$$

where $bG_i$ is glucose value on $i^{th}$ day, where index i is 1, 2, 3, ...L, from the latest glucose measurement to oldest glucose measurement. Similarly, for a cohort aged L-1 day, mean bG can be defined according to Equation (31) as:

$$\frac{\sum_{i=1}^{L-1} bG_i \Delta T}{\sum_{i=1}^{L-1} \Delta T} = \frac{1}{L-1} \sum_{i=1}^{L-1} bG_i \qquad (31).$$

And so on. Collecting weights for same $bG_i$, the weights may be defined according to Equation (32) as:

$$\left[ \left(\frac{1}{L}\right) bG_L \quad \left(\frac{1}{L} + \frac{1}{L-1}\right) bG_{L-1} \quad \dots \quad \left(\frac{1}{L} + \frac{1}{L-1} + \dots + \frac{1}{1}\right) bG_1 \right] \qquad (32).$$

It is to be appreciated that the above weighting scheme corresponds to a harmonic series. As such, the weights will be referred to herein as harmonic weighting.

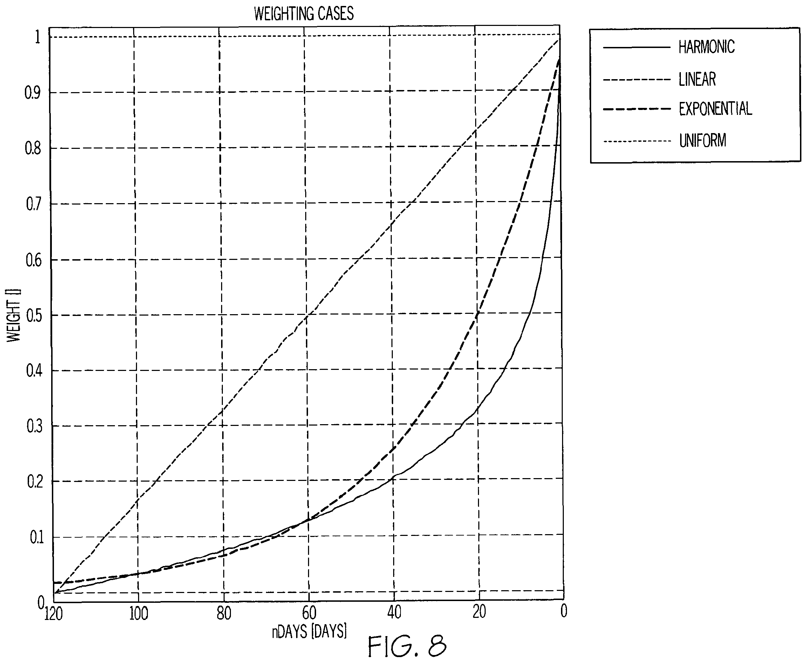

FIG. 8 shows 4 weighting schemes that were considered in developing the prediction model for HbA1C. From using the harmonic weighting in the above Equation (32), it is clear that older bG values contribute progressively less and less to HbA1C value. If the area under the harmonic curve is considered, then the period covering 60 days represents 84.4% of the total, which is shown in Table 3.

**Table 3: Area under the harmonic curve**

| Visitation Period | Percentage Area |
| --- | --- |
| From=1 To=1 | 0 |
| From=10 To=1 | 28.1 |
| From=20 To=1 | 45.7 |
| From=30 To=1 | 59.0 |
| From=40 To=1 | 69.5 |
| From=50 To=1 | 77.8 |
| From=60 To=1 | 84.4 |
| From=70 To=1 | 89.6 |
| From=80 To=1 | 93.6 |
| From=90 To=1 | 96.5 |
| From=100 To=1 | 98.5 |
| From=110 To=1 | 99.6 |

(continued)

| Visitation Period | Percentage Area |
|---|---|
| From=120 To=1 | 100 |

Additional results showed that harmonic temporal weighting is a relevant scheme in the determination of the HbA1C estimate based on SMBG measurements, and that the period over which SMBG data contributes significantly to estimating HbA1C is about 60 days (considering in this case life of erythrocytes as 120 days. Similar reasoning can be used when considering erythrocytes for other ages). Analysis results also supports that collecting bG values collected over a visitation period of about approximately 60 days provides the best estimate on HbA1C. In one embodiment, the collecting of both bG measurements and associated context of the bG measurement at daily times specified by the structured sampling schema is over a period of about 2 to about 4 months. In another embodiment, a small time window such as ranging from 1 week to 4 weeks can be used as a representative of glucose behavior covering a 3 to 4-month period. In other embodiments, a short period i.e., less than 7 days, can be used to provide a prediction/indication of a patient's probable HbA1C and/or of the effect an action is having on a patient's HbA1C, via the change indicated by the estimated HbA1C. Such a prediction, for example, may be provided using the data collected from a focused or structured collection (testing) procedure (such as described in U.S. Patent Application Serial No. 12/643338), and in particular, those procedures that may take, e.g., less than 7 days to conduct. For example, in one particular embodiment, a structured testing procedure is provided in which seven spot measurements are requested, e.g., before each meal, 2 hours after each meal, and before going to bed, for 3 consecutive days, and after which the estimated HbA1C is computed and provided as a prediction of the probable HbA1c level of the patient. Such a structured testing procedure is also useful in situations when there is a need to reveal particular blood glucose peaks and blood glucose minimums of interest, especially in the case of patients who do not receive any insulin. It is to be appreciated that the above described embodiments can allow the HCP and the patient to revise the current therapy or behavior to try achieve prescribed targeted goals. Furthermore, it is to be appreciated that providing such a prediction/indication of a patient's probable HbA1C and/or of the effect an action is having on the patient's HbA1C from an estimated HbA1C resulting from any of the above described embodiments can be used in therapy control much earlier than having to wait 3 months in order to obtain the actual HbA1c value to see whether a drug, such as e.g., GLP1, is efficient or not, and thereby potentially save time for such therapy control. Accordingly, it is to appreciated that the resulting predicted HbA1C then represents a future HbA1C, which can provide the patient and/or HCP the future glycemic level of the patient (i.e., assuming the current glucose behavior is maintained). The principles behind the process used to derive a correlation coefficient for meal segments according to the present invention is now discussed hereafter.

*Correlation coefficient for meal segments*

Glucose data collected during two independent clinical studies in 2003 and 2006 were used to determine a correlation coefficient for meal segments from which to devise a sampling schema for use with the HbA1C prediction model. The clinical trials studied the post prandial glucose control for meals with different meal composition. The key aspects of each of the two studies are summarized below.

*Meal study 2003*

1. Study was conducted during 2003-2004. The study was designed to examine the meal response of fixed insulin bolus to meals with varying glucose absorption characteristics.
2. Demographics of the subjects participating in the study are:

    a. Number of Subjects=23
    b. Number of study blocks=4
    c. Number of males=12, Number of females=11
    d. Age (40±9) years
    e. Weight (75±15) kg
    f. BMI (24.6±2.5) kg/m$^2$
    g. HbA1C (7.0±1.0)%

3. Each visit is 4 days long:

a. Day 1:

    i. Subject arrives in the evening to be instrumented.
    ii. Has an evening supper
    iii. Spot monitoring

b. Day 2:

    i. 9 am Test meal (A, B, C, D, E and F)
    ii. 3 pm late lunch meal
    iii. 7 pm supper

c. Day 3:

    i. 9 am Test meal (A, B, C, D, E and F)
    ii. 3 pm late lunch meal
    iii. 7 pm supper

d. Day 4:

    i. Subject leaves around breakfast time

4. Number of study blocks is 4. A study block is the re-visitation of the subject for performing the meal study with a different test meal and/or insulin therapy algorithm.
5. Meal segments were extracted from Meal Study 2003. The segments were of duration:

    a. 6 hr, all test meals (@ 9:00 am)
    b. 4 hr, all late lunch (@ 3:30 pm)
    c. 8 hr, all supper (@ 7:00 pm)

*Meal study 2006*

1. Study was conducted during the year 2006-2007
2. Demographics of the subjects:

    a. Number of Subjects=12
    b. Number of study blocks=4
    c. Number of males=7, Number of females=5
    d. Age ($45\pm9$) years
    e. Weight ($75\pm14$) kg
    f. BMI ($24.7\pm3.0$) kg/m$^2$
    g. HbA1C ($6.9\pm0.8$)%

3. Each visit (block) is 4 days long:

    a. Day 1:

        i. Subject arrives in the evening to be instrumented.
        ii. Has a evening supper
        iii. Spot monitoring

    b. Day 2:

        i. 9 am Test meal (A, B, E and F)
        ii. 3 pm late lunch meal
        iii. 7 pm supper

c. Day 3:

> i. 9 am Test meal (A, B, E and F)
> ii. 3 pm late lunch meal
> iii. 7 pm supper

d. Day 4:

> i. Subject leaves around breakfast time

4. Number of study blocks is 4. A study block is the re-visitation of the subject for performing the meal study with a different test meal and/or insulin therapy algorithm.

5. Meal segments were extracted from Meal Study 2006. The segments were of duration:

> a. 6 hr, all test meals (@ 9:00 am)
> b. 4 hr, all late lunch (@ 3:30 pm)
> c. 8 hr, all supper (@ 7:00 pm)

The above test meal labels A-F describe the meal speed. Meals labeled A and B are fast meals, meals labeled C and D are regular, and meals labeled E and F are slowly absorbing meals. The meals were classified by a professional dietician. The meal study data set provided discrete frequently measured bG data, where the sampling rates for the time window covering the test meals were 10 minutes. Sampling rates at other times range from 1 minute to as rarely as hourly measurement, such as overnight. Also available in the bG data is specific insulin, ingested mixed meal information and interventions. It is to be appreciated that the clinical bG data set did not include HbA1C values. HbA1C values were then generated artificially by using the Mortensen model (Equations (1)-(3)) with the bG data.

It is clear from earlier analysis that there exists a linear relationship between true mean bG and HbA1C. It is then clear that one could simply focus on the question of determining either true mean bG or HbA1C. Given the continuous and/or frequent bG measurements in the bG data, the bG curves were then segmented into relevant groups and correlation between various parameters such as minimum, maximum, glucose value at specified time and so forth were correlated to true mean bG as well as HbA1C.

In regards to HbA1C, this value was determined by inputting the glucose curve to the Mortensen model Equations (1)-(3). In this regard then, the meal data was first divided into meal segments. Each of the meal segment was curve fitted and then the resulting signal was repeated to create as input a bG input signal of duration 150 days. The resulting profile was then passed through the Mortensen model. (Equations (1)-(3)) to generate the HbA1C values. In this way HbA1C for each of the meal segments was generated.

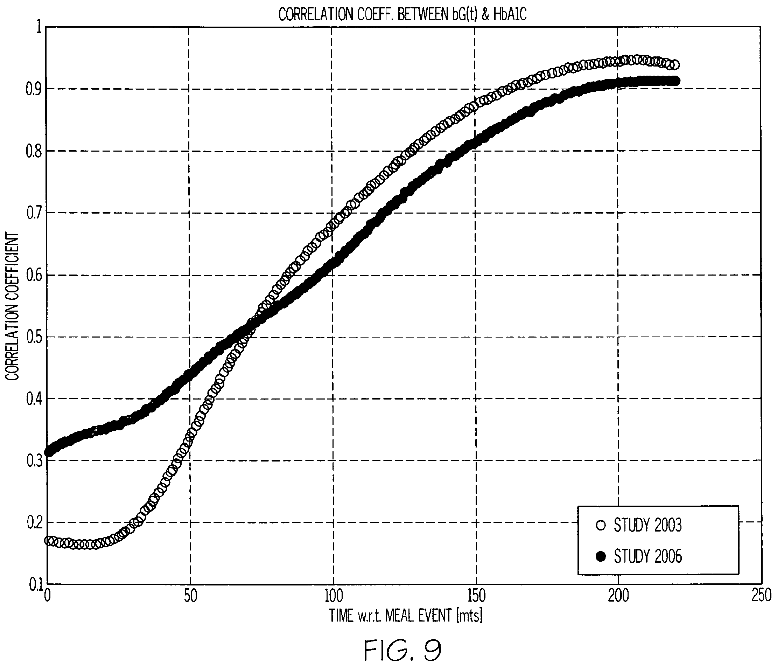

Next, several predictors were examined to correlate with HbA1C. The most meaningful predictor discovered by the inventors was a bG measurement taken at a particular post-prandial time point. For this predictor, a Pearson correlation coefficient was used as a function of bG(t) which is shown plotted in FIG. 9. The correlation coefficient for meal segments extracted from the clinical meal studies of 2003 and 2006 shows that there is a strong linear relationship between HbA1C and post prandial bG measurement when $t \geq 150$ minutes.

Although the correlation coefficients may differ for different clinical studies, in general the trends are expected to be similar. As shown by FIG. 9, a low correlation is seen in the 1st hour; the correlation then starts increasing and reaches values greater than 0.8 for 2.5 hrs postprandial. Such variation could be explained by meal type, meal amount and associated insulin therapy. Low correlation in early hours of post prandial is due to transients caused by variations due to meal glucose absorption and due to insulin absorption characteristics. As the transients die out the correlation increases. The increased correlation for the clinical studies is also due to following reasons: the subjects are well motivated, so in general, their glycemic excursion should recover quite consistently for subjects during postprandial period.

The variations in meal behavior are due to main factors such as physiology, meal content variation, inaccuracies in physiological parameter estimates, basal setting. The correlation coefficients indicate that meals correlate to HbA1C very strongly when bG measurements are conducted postprandially in time range around 3 hours. It is also clear from simulation that the transient bG has comparatively less impact than the steady state behavior of the meal that is the relatively slow and steady push. The variability in the early transients is clearly indicative of lack of specific knowledge of day to day physiological variability and imprecise knowledge of meal but the general control strategy on the latter post prandial state is important in achieving low HbA1C.

The following section hereafter focuses on deriving an optimal sampling schema for determination of true mean bG and HbA1C. Sampling schema is determined by using the equations developed in earlier sections, such as lifestyle related time weighting addressing a modal day, and glucose weighting addressing the data covering a visitation period (i.e., period between visitations).

*Structured Sampling Schema*

Using clinical data from Meal study 2003, bG profiles are generated by combining various meal segments by randomly selecting bG profile segments from different meal bins and concatenating the segments. The various meal bins are listed in Table 4.

**Table 4: Meal Bins**

| | Breakfast | Lunch | Supper + Overnight |
|---|---|---|---|
| **Low - HbA1C** | First 1/3rd of ranked Breakfast meals | First 1/3rd of ranked lunch meals | First 1/3rd of ranked supper meals |
| **Medium - HbA1C** | Second 1/3rd of ranked Breakfast meals | Second 1/3rd of ranked lunch meals | Second 1/3rd of ranked supper meals |
| **High - HbA1C** | Third 1/3rd of ranked Breakfast meals | Third 1/3rd of ranked lunch meals | Third 1/3rd of ranked supper meals |

The bins in Table 4 represent meal segments and are first of all grouped by collecting the segments obtained from breakfast, lunch and supper and overnight time periods. The meal segments were further ranked and sorted in ascending order in terms of corresponding HbA1C values from simulation. The breakfast meal pool was then divided into 3 equal groups by selecting the first one third breakfast meals and labeled as low - HbA1C, then the second one third of breakfast meals labeled as Medium - HbA1C and the remaining breakfast meals as High - HbA1C. In a similar fashion lunch and supper are also binned. In all, 9 meal bins were created. To create lifestyle based bG sequence, lifestyle is described as the modal day consisting of breakfast starting at 8 am with one of the HbA1C group (Low, Medium or High); lunch at noon with one of the HbA1C group (Low, Medium or High) and supper at 6 pm with one of the HbA1C group (Low, Medium or High). In this manner 174 bG sequences were generated covering various combinations.

As mentioned in the previous section, if bG measurements are conducted postprandially around the time interval when the correlation coefficient is high (e.g., t≥150 minutes, FIG. 9) a good estimate of HbA1C can be anticipated. Therefore, the key factors relating to a useable prediction of HbA1C from a series of bG measurements are the following: (a) timing of bG measurement, (b) number of bG measurements (in range of 2-6 measurements per day), (c) accuracy of predicted A1C, and (d) bias of the predicted A1C.

As per lifestyle (primarily done for meal in the illustrated embodiment) the sampling schema was setup according to Table 5 as follows:

**Table 5: Sampling Schema setup**

| | |
|---|---|
| Center of the Sampling window (WinCen) | Determine the optimal expected time at which one should sample for SMBG. |
| Size of the sampling window (WinSize) | The allowance/tolerance to measurement time window around WinCen. |
| Number of Days (nDays) | SMBG data is collected over period of last nDays days. |
| Number of samples (nSamples) | The bG sampling is event driven. With respect to each meal event the number of samples collected during the specified number of days, nDays. As an example nSamples=50 means that as described by Lifestyle (FIG. 5) for breakfast there are 50 bG measurements spanning over nDays, then 50 measurements for lunch spanning the nDays and then for supper 50 measurements spanning the nDays. |
| Sampling Ratio, $\dfrac{nSamples}{nDays}$ | It is the ratio of number of glucose measurements to the number of days (nDays) over which the samples are collected, for each event type. For example, nSamples=50 and nDays=70 then Sampling ratio=50/70. |

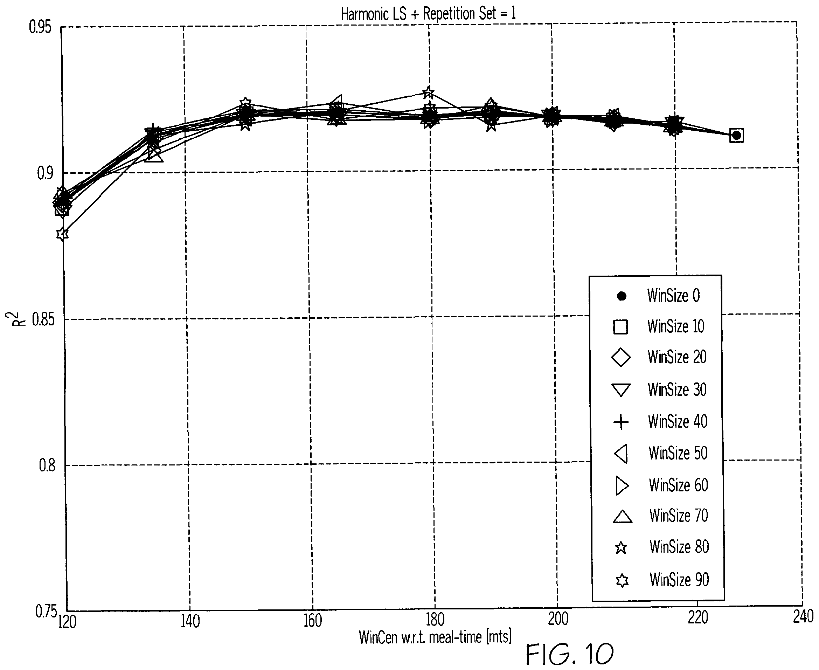

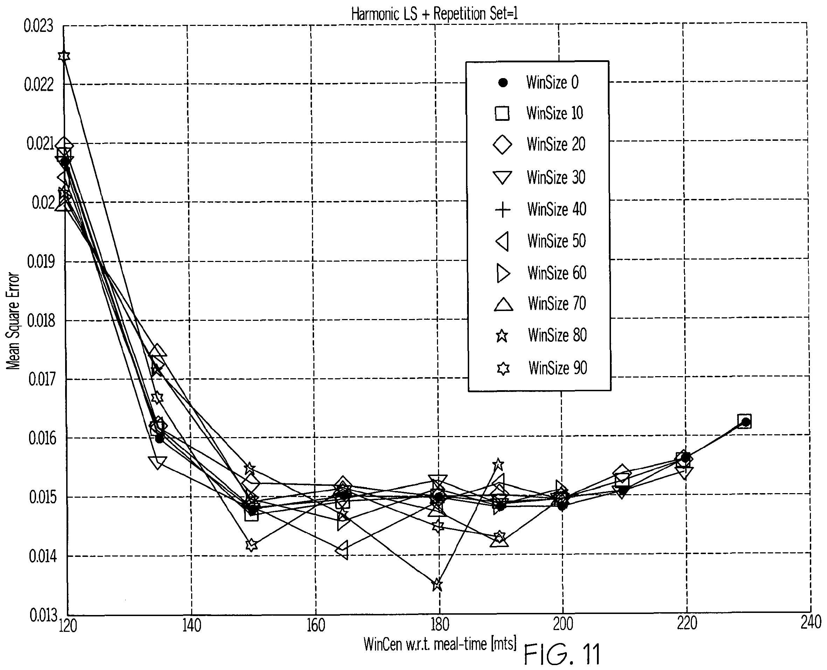

Linear regression was then carried out to predict HbA1C from SMBG measurements, whereby SMBG values were processed by various lifestyle weighting and averaging strategies. FIG. 10 shows the $R^2$ (R-squared value) from the linear regression against HbA1C for various values of WinCen and WinSize for the lifestyle. For the illustrated plot of FIG. 10, the visitation period (nDays) equals 60 days, and the number of samples (nSamples) also equals 60. As shown, the best $R^2$ is centered on post-prandial time of 190 minutes. Similarly plotting the results for mean squared error (FIG. 11) it is observed that a postprandial measurement around 180 minutes provides minimum error in HbA1C estimate.

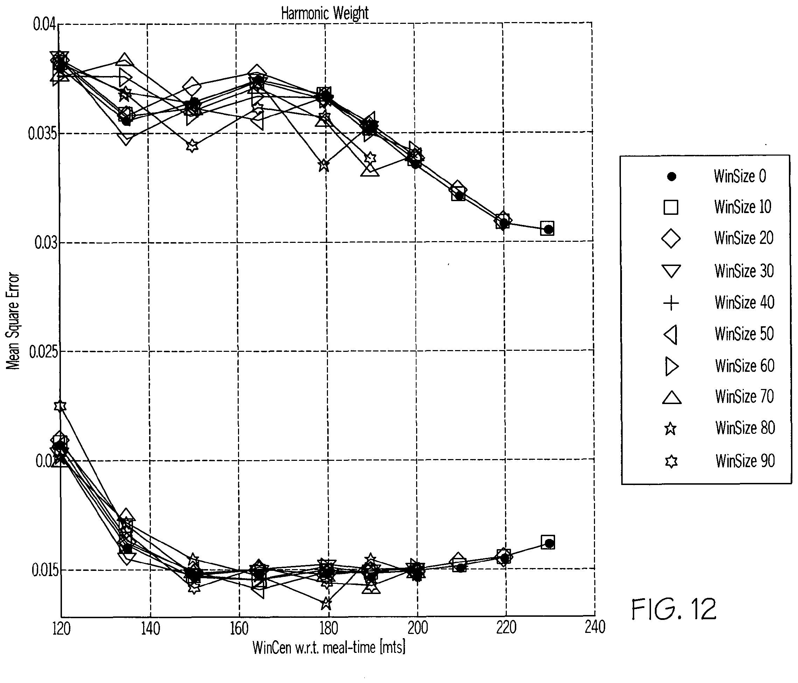

In FIG. 12, a comparison between the daily lifestyle weighting and no daily lifestyle weighting shows that daily life style weighting produces lower MSE (mean squared error).

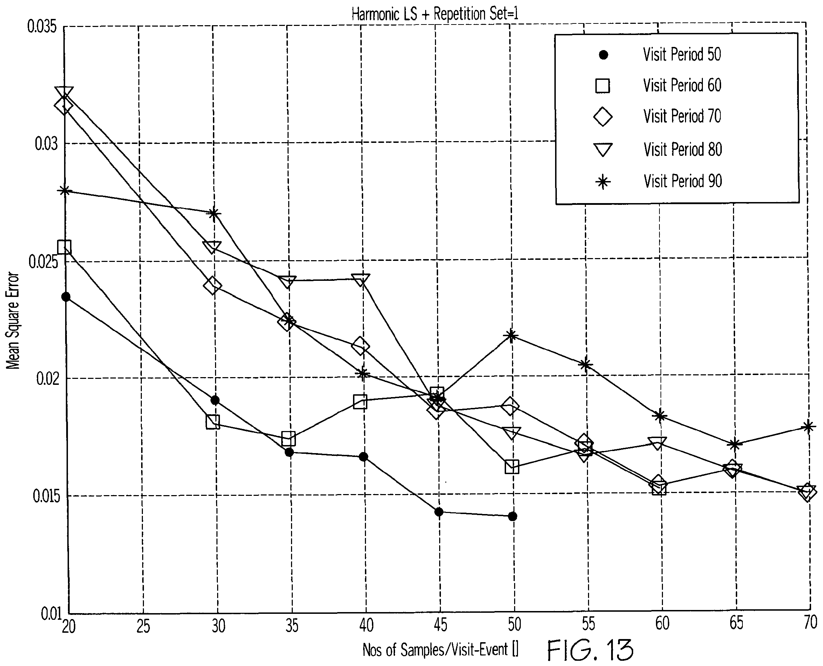

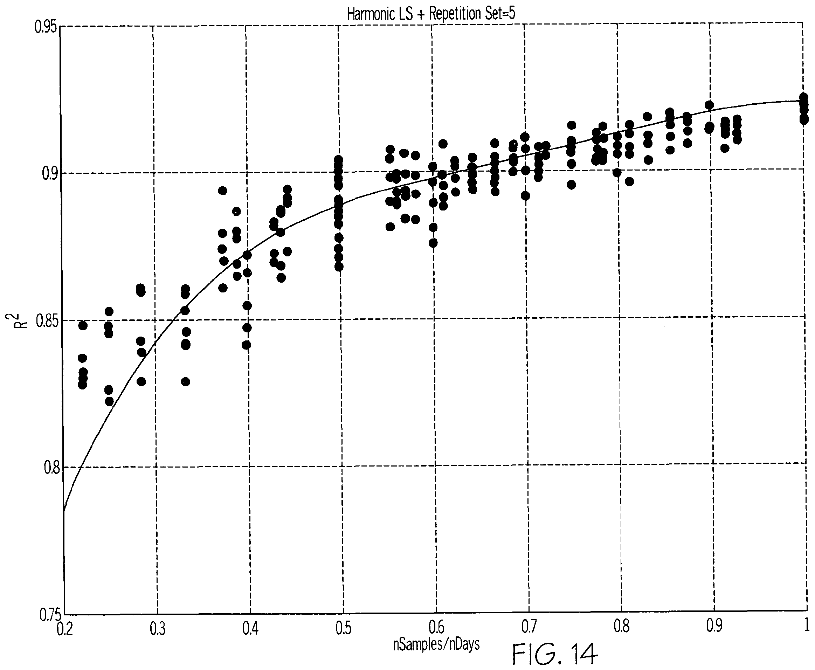

FIG. 13 shows the impact of nDays (visitation period) and nSamples for WinCen of 190 minutes and WinSize of 50 minutes. It shows that MSE reduces as the number of SMBG measurements is increased. In particular, there is an impact of nDays. The number of days shows that there is an optimal number of days beyond which the $R^2$ does not improve. To determine the best nSamples and nDays one actually needed to look at the sampling ratio which is the number of samples per event (nSamples/nDays). The requirement was to have the ratio as small as possible with some acceptable $R^2$. What was observed was that below 0.5 both $R^2$ deteriorate at a rapid pace and also the spread in their values became greater. For a sampling ratio greater than 0.5 and above, the $R^2$ value was > 0.85. For $R^2 > 0.9$, a sampling

ratio $\dfrac{nSamples}{nDays}$ of 0.55 was obtained as shown by FIG. 14.

*Regression Model for estimating HbA1C*

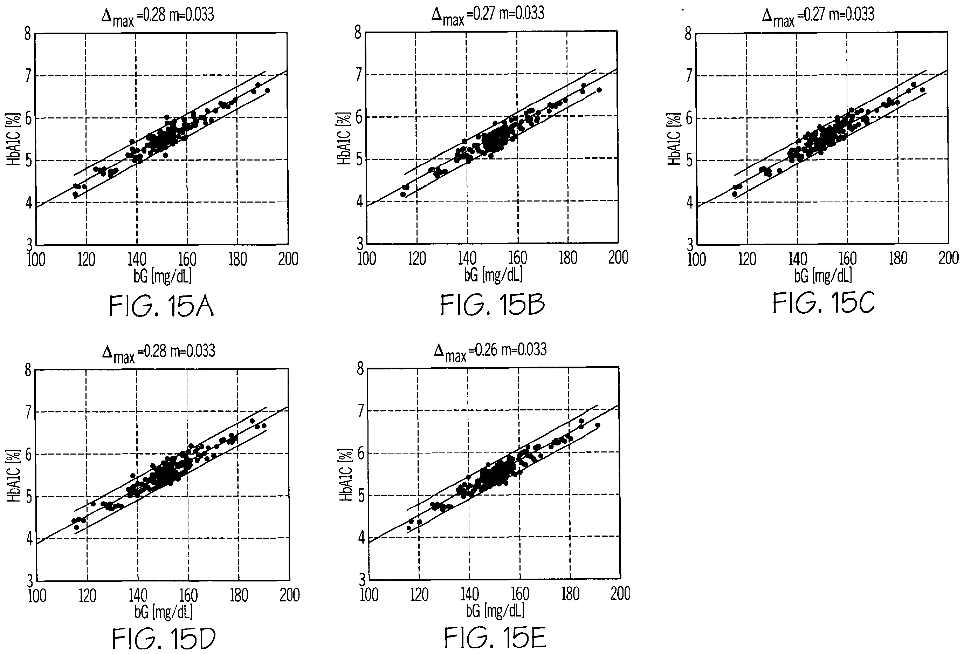

As per the sampling schema, the sampled bG data were then regressed and plotted, which are shown by FIGS. 15A-E. Tabulated results of the regression are provided in Table 6, which shows that the parameters for each linear regression are in close proximity to each other. In FIGS. 15A-E, the prediction line (center line) and a 95% confidence interval (CI) boundaries (above and below curves) are shown in the subplots. The 95% CI covers a range which deviates approximately 0.26% HbA1C from the nominal value.

**Table 6: Linear regression parameters**

| Figure | Delta | Slope | Intercept |
|--------|-------|-------|-----------|
| 15A | 0.28 | 0.033 | 0.587 |
| 15B | 0.27 | 0.033 | 0.581 |
| 15C | 0.27 | 0.033 | 0.588 |
| 15D | 0.28 | 0.033 | 0.547 |
| 15E | 0.26 | 0.033 | 0.548 |

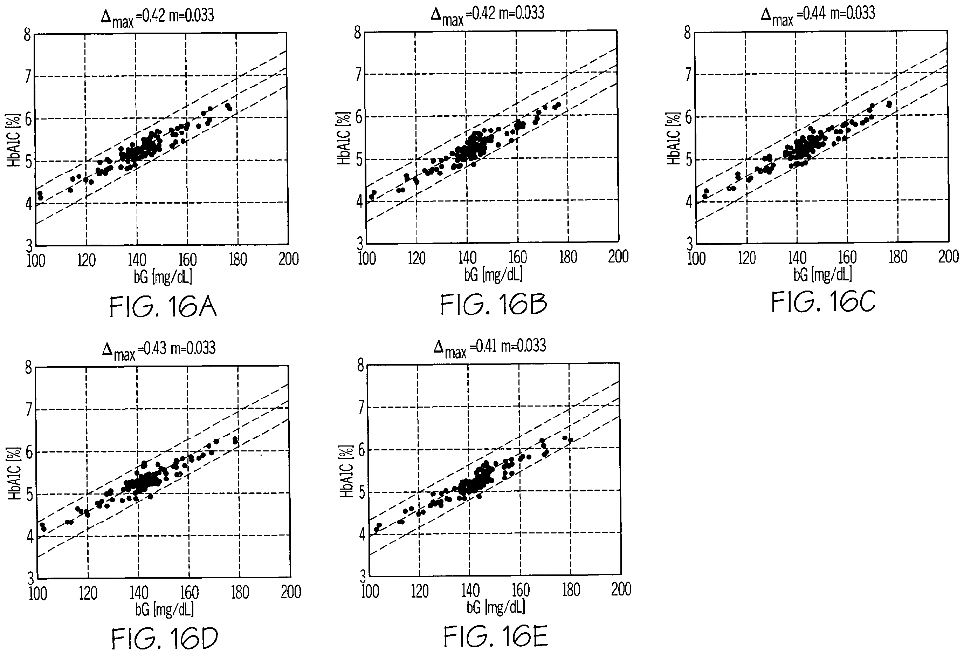

FIGS. 16A-E show that CI boundaries contain almost all of the HbA1C observations. A slope of 0.033 or 1/30 is obtained from the linear regression. In summary, the optimal SMBG sampling parameters along with regression parameters for determining an estimated true mean bG value and estimated HbA1C value is listed in Table 7.

**Table 7: Structured Sampling Schema**

| Parameter | Optimal value |
|-----------|---------------|
| WinCen | 190 min |
| WinSize | 50 min |
| nSamples | 45 samples per event |
| nDays | 80 days |
| Weighting function | Harmonic |
| Lifestyle weighting | Yes |
| Estimated HbA1C | $0.033\overline{bG} + 0.5702$ |

*Validation*

To validate the results obtained above in Table 7, which were derived using the meal segments extracted from the Meal Study 2003, the Meal Study 2006 was then used. Similar to Meal Study 2003, all the meal segments from Meal Study 2006 were extracted. Overall, 286 meal segments were obtained from the 2006 study. All the meal segments were then fitted by a polynomial curve, and ordered in an ascending order by their individual HbA1C values (obtained by using the Mortensen Model). The meal segments were then binned into groupings done in the manner explained for Meal Study 2003. Using the meal segments, a bG sequence covering a duration of 300 days was generated as per the previous lifestyle used in the 2003 meal study. The simulation duration was also set to 300 days. The bG profiles and HbA1C were then stored for sampling and HbA1C prediction. In all 108 simulations were generated.

The bG values were then sampled as per the Sampling Schema listed in Table 7. Using the sampled bG values for each of the 108 simulation cases, mean bG was determined. The relation between the mean bG and HbA1C, as determined by simulation, is plotted in FIGS. 16A-E. Each of the FIGS. 16A-E is a subplot which simply repeats a random sampling as per the schema explained earlier. Each subplot shows the estimated mean bG with the true HbA1C. The upper and lower lines in each subplot indicated 3 standard deviations (SD line) from the predicted HbAC1 algorithm, $0.033\overline{bG} + 0.5702$, (e.g., center line in each subplot) as determined previously above. It is to be appreciated that the distance between the SD line and the mean behavior on an average is 0.44 % HbA1C. Therefore, as expected there was a degradation (spread) in the estimated HbA1C value, however the resulting precision was still within 3% CV.

From the above results, if a slightly lower $R^2$ of 0.85 is used, then the number of measurements/event can be reduced to 45 over 80 days. With 3 meal events per day plus a night time measurement, then the number of measurements are equal 180 measurements. This implies approximately 2.25 measurements/day are needed as per sampling schema described above to achieve an estimated HbA1C value that has a precision within 3% CV.

*Implementation examples*

The above described sampling schema and prediction algorithm for providing both an estimated true mean blood glucose value and an estimated glycated hemoglobin (HbA1C) value from structured spot measurements of blood glucose may be implemented using hardware, software or a combination thereof. For example, the above described sampling schema and prediction algorithm may be implemented in one or more microprocessor based systems, such as a portable computer or other processing systems, such as personal digital assistants (PDAs), or directly in self-monitoring glucose devices or meters (bG meters) equipped with adequate memory and processing capabilities to process a chronological sequence of measurements of a time dependent parameter measured in or on the human body, namely of the glucose level (e.g. the glucose (bG) level).

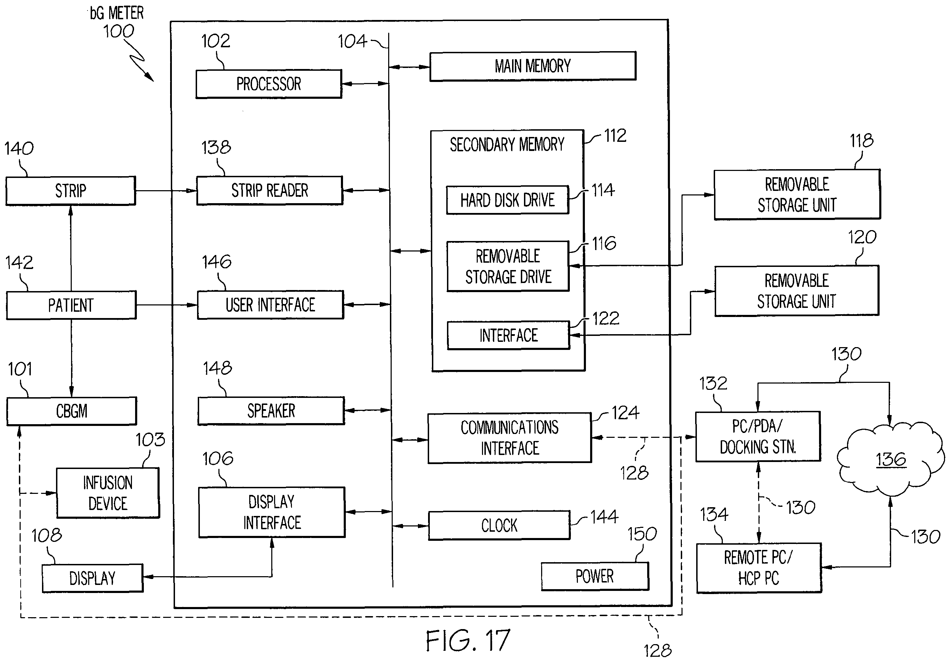

In an example embodiment, the sampling schema and prediction algorithm are implemented in software running on a self-monitoring blood glucose (bG) meter 100 as illustrated in FIG. 17. The bG meter 100 is common in the industry and includes essentially any device that can function as a glucose acquisition mechanism. The bG meter 100 or acquisition mechanism, device, tool, or system includes various conventional methods directed toward drawing a sample (e.g. by finger prick) for each test, and making a spot determination of the glucose level using an instrument that reads glucose concentrations by optical, electrochemical, electromechanical or calorimetric detection/measurement methods. In addition, the bG meter 100 may include indwelling catheters and subcutaneous tissue fluid sampling devices and/or communicate with devices, such as continuous glucose monitor (CGM) 101, having indwelling catheters and subcutaneous tissue fluid sampling devices, and/or a drug pump/infusion device 103.

In the illustrated embodiment, the bG meter 100 includes one or more microprocessors, such as processor 102, which is connected to a communication bus 104, which may include data, memory, and/or address buses. The bG meter 100 may include a display interface 106 providing graphics, text, and other data from the bus 104 (or from a frame buffer not shown) for display on a display 108. The display interface 106 may be a display driver of an integrated graphics solution that utilizes a portion of main memory 110 of the meter 100, such as random access memory (RAM) and processing from the processor 102 or may be a dedicated graphics card. In another embodiment, the display interface 106 and display 108 additionally provide a touch screen interface for providing data to the bG meter 100 in a well known manner.

Main memory 110 in one embodiment is random access memory (RAM), and in other embodiments may include other memory such as a ROM, PROM, EPROM or EEPROM, and combinations thereof. In one embodiment, the bG meter 100 includes secondary memory 112 which may include, for example, a hard disk drive 114 and/or a removable storage drive 116, representing a floppy disk drive, a magnetic tape drive, an optical disk drive, a flash memory, etc. The removable storage drive 116 reads from and/or writes to a removable storage unit 118 in a well known manner. Removable storage unit 118, represents a floppy disk, magnetic tape, optical disk, flash drive, etc. which is read by and written to by removable storage drive 116. As will be appreciated, the removable storage unit 118 includes a computer usable storage medium having stored therein computer software and/or data.

**EP 2 445 394 B1**

In alternative embodiments, secondary memory 112 may include other means for allowing computer programs or other instructions to be loaded into the bG meter 100. Such means may include, for example, a removable storage unit 120 and an interface 122. Examples of such removable storage units/interfaces include a program cartridge and cartridge interface, a removable memory chip (such as a ROM, PROM, EPROM or EEPROM) and associated socket, and other removable storage units 120 and interfaces 122 which allow software and data to be transferred from the removable storage unit 120 to the bG meter 100.

The bG meter 100 in one embodiment includes a communications interface 124. The communications interface 124 allows software and data to be transferred between the bG meter 100 and an external device(s) 132. Examples of communications interface 124 may include one or more of a modem, a network interface (such as an Ethernet card), a communications port (e.g., USB, firewire, serial or parallel, etc.), a PCMCIA slot and card, a wireless transceiver, and combinations thereof. In one embodiment, the external device 132 is a personal computer (PC), and in another embodiment is a personal digital assistance (PDA). In still another embodiment, the external device 132 is a docking station wherein the communication interface 124 is a docket station interface. In such an embodiment, the docking station may provided and/or connect to one or more of a modem, a network interface (such as an Ethernet card), a communications port (e.g., USB, firewire, serial or parallel, etc.), a PCMCIA slot and card, a wireless transceiver, and combinations thereof. Software and data transferred via communications interface 124 are in the form of wired or wireless signals 128 which may be electronic, electromagnetic, optical, or other signals capable of being sent and received by communications interface 124. For example, as is known, signals 128 may be sent between communication interface 124 and the external device(s) 132 using wire or cable, fiber optics, a phone line, a cellular phone link, an RF link, an infrared link, other communications channels, and combinations thereof.

In one embodiment, the external device 132 is used for establishing a communication link 130 between the bG meter 100 and still further electronic devices such as a remote Personal Computer (PC) of the patient, and/or a health care provider (HCP) computer 134, directly or indirectly, such as through a communication network 136, such as the Internet and/or other communication networks. The communication interface 124 and/or external device(s) 132 may also be used to communicate with further data gathering and/or storage devices such as insulin delivering devices, cellular phones, personal digital assistants (PDA), etc. Specific techniques for connecting electronic devices through wired and/or wireless connections (e. g. USB and Bluetooth, respectively) are well known in the art.

In the illustrative embodiment, the bG meter 100 provides a strip reader 138 for receiving a glucose test strip 140. The test strip 140 is for receiving a sample from a patient 142, which is read by the strip reader 138. Data, representing the information provided by the test strip, is provided by the strip reader 138 to the processor 102 which executes a computer program stored in memory 110 to perform various calculations as discussed in great detail below on the data. The results of the processor 102 from using the data is displayed on the display 108 and/or recorded in secondary memory 112, which is herein referred to as self monitored glucose (bG) data. The bG data may include, but not limited thereto, the glucose values of the patient 142, the insulin dose values, the insulin types, and the parameter values used by processor 102 to calculate future glucose values, supplemental insulin doses, and carbohydrate supplements. Each glucose value and insulin dose value is stored in memory 112 with a corresponding date and time. An included clock 144 of the bG meter 100 supplies the current date and time to processor 102. The bG meter 100 further provides a user input device(s) 146 such as keys, touchpad, touch screen, etc. for data entry, program control, information requests, and the likes. A speaker 148 is also connected to processor 102, and operates under the control of processor 102 to emit audible and/or visual alerts/reminders to the patient of daily times for bG measurements and events, such as for example, to take a meal, of possible future hypoglycemia, and the likes. A suitable power supply 150 is also provided to power the bG meter 100 as is well known to make the meter portable.

The terms "computer program medium" and "computer usable medium" are used to generally refer to media such as removable storage drive 116, a hard disk installed in hard disk drive 114, signals 128, etc. These computer program products are means for providing software to bG meter 100. The invention includes such computer program products.

Computer programs (also called computer control logic) are stored in main memory 110 and/or secondary memory 112. Computer programs may also be received via the communications interface 124. Such computer programs, when executed, enable the bG meter 100 to perform the features of the present invention as discussed herein. In particular, the computer programs, when executed, enable processor 102 to perform the functions of the present invention. Accordingly, such computer programs represent controllers of bG meter 100.

In an embodiment where the invention is implemented using software, the software may be stored in a computer program product and loaded into bG meter 100 using removable storage drive 116, removable storage unit 120, hard disk drive 114, or communications interface 124. The control logic (software), when executed by the processor 102, causes the processor 102 to perform the functions of the invention as described herein.

In another embodiment, the invention is implemented primarily in hardware using, for example, hardware components such as application specific integrated circuits (ASICs). Implementation of the hardware state machine to perform the functions described herein will be apparent to persons skilled in the relevant art(s).

In yet another embodiment, the invention is implemented using a combination of both hardware and software.

In an example software embodiment of the invention, the methods described hereafter are implemented in the C++ programming language, but could be implemented in other programs such as, but not limited to, Visual Basic, C, C#, Java or other programs available to those skilled in the art (or alternatively using script language or other proprietary interpretable language used in conjunction with an interpreter).

As mentioned above, the bG meter 100 is used by the patient 142 for recording, *inter alia*, insulin dosage readings and spot measured glucose levels. Such bG data obtained by the bG meter 100 in one embodiment is transferable via the communication interface 124 to another electronic device, such the external device 132 (PC, PDA, or cellular telephone), or via the network 136 to the remote PC and/or HCP computer 134. Examples of such bG meters include but are not limited to, the Accu-Chek Active meter and the Accu-Chek Aviva system both by Roche Diagnostics, Inc. which are compatible with the Accu-Chek 360° Diabetes management software to download test results to a personal computer or the Accu-Chek Pocket Compass Software for downloading and communication with a PDA. The program may run on a remote server and generate result. The result is available by one or more communication mode(s) stated earlier. The program device is also functional with 3rd party devices which communicate with 132, 134

Accordingly, it is to be appreciated that the bG meter 100 includes the software and hardware necessary to process, analyze and interpret the self-recorded diabetes patient (i.e., bG) data in accordance with predefined flow sequences (as described below in detail) and generate an appropriate data interpretation output. In one embodiment, the results of the data analysis and interpretation performed upon the stored patient data by the bG meter 100 are displayed in the form of a report, trend-monitoring graphs, and charts to help patients manage their physiological condition and support patient-doctor communications. In other embodiments, the bG data from the bG meter 100 may be used to generated reports (hardcopy or electronic) via the external device 132 and/or personal computer (PC) and/or HCP computer 134.

The bG meter 100 further provides the user and/or his or her HCP with the possibilities of a) editing data descriptions, e. g. the title and description of a record; b) saving records at a specified location, in particular in user-definable directories as described above; c) recalling records for display; d) searching records according to different criteria (date, time, title, description etc.); e) sorting records according to different criteria (values of the bG level, date, time, duration, title, description etc.); f) deleting records; g) exporting records; and/or h) performing data comparisons, modifying records, excluding records as is well known.

As used herein, lifestyle is described in general as a pattern in an individual's habits such as meals, exercise, and work schedule. The individual additionally may be on medications such as insulin therapy or orals that they are required to take in a periodic fashion. Influence of such action on glucose is implicitly considered by the present invention.

*Estimating True mean bG and HbA1C*

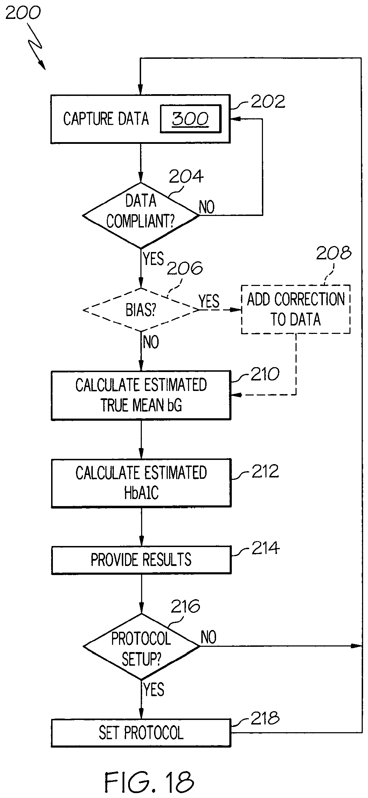

With reference made also to FIG. 18, a method 200 according to one embodiment of the present invention is described. In step 202, bG (i.e., spot) measurements of the patient 142 is captured. In one embodiment, each of the bG spot measurements is captured via strip 140 provided with a sample of the patient's which is then in turn read by a strip reader and analyzed by processor 102 to give the bG measurement of the patient 142. In other embodiments, the bG measurements may be captured at times dictated by the continuous glucose monitor 101 and/or commanded by the patient. As is well know the result of a newly taken bG measurements is displayed to the patient on display 108 as well as stored such as, for example, in memory 112 together with a time (e.g., GMT) and date of the measurement, via processor 102 reading clock 144 in step 202.

In one embodiment and generally, as mentioned above the bG meter 100 stores the results of the glucose (bG) measurements in its memory 112 together with a date-time stamp and associated event information to create a chronological sequence or set G of bG spot measurements, such as measurements $bG_1^k$, $bG_2^k$, $bG_3^k$, $bG_4^k$, and $bG_5^k$, where *k* is the day. The measurement set *G* is sorted by increasing time and may span several days. In one embodiment, the date stored in memory with the measurement consists of some representation of day/month/year, and the time consists of some representation of the time of day (e.g. hh:mm:ss). In other embodiments, other date and time recording methods may be used, such as for example, using a Julian calendar and an alternative count interval for time.

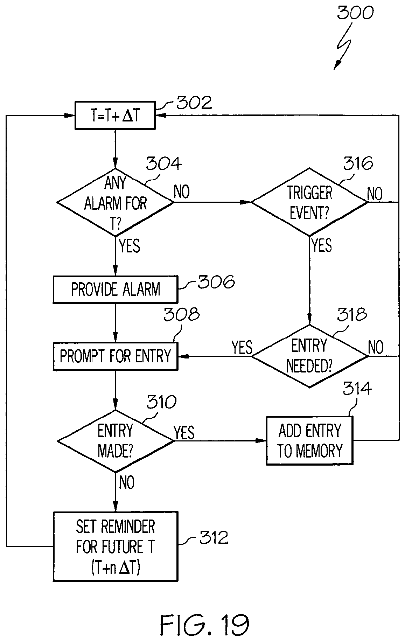

Along with each bG measurement, the patient is requested to input event information concerning the patient's lifestyle. In one embodiment, the meter 100 has enough memory to maintain bG data for at least 60 days with the associated event information concerning the patient's lifestyle. In one embodiment, lifestyle is classified by information concerning the following events: breakfast, lunch, supper, snack, exercise, physical activity, stress, and optionally any other relevant event that is custom set into the meter. As with the bG measurements, such events are time stamped and associated with a description of event such as, for example, magnitude, intensity, duration, etc. Other such event characterizations are described more fully in commonly owned and co-pending U.S. application Ser. Nos. 11/297,733 and 12/119,201, which are herein incorporated by reference. Manual input of the event description by the patient in one embodiment is driven by a questionnaire presented to the patient on the meter 100. In one embodiment, the questionnaire is provided by an HCP or designed to be set up by the patient according to provided instructions contained on the meter 100. In another embodiment, the meter 100 is provided with scheduled reminders (i.e. alarms) which are provided at particular time in order to record such event information, e.g., via the questionnaire, within the compliance window according to the sampling schema of Table 7. An event scheduler 300 (FIG. 19) may be provided for this purpose and executed by the processor 102 of the meter 100, which an example thereof is discussed hereafter.

FIG. 19 depicts a process of the event scheduler 300. In step 302, a timer T synced with the clock 144 is incremented wherein in step 304, the processor 102 checks a structured sampling schema, such as included in a protocol file provided in memory 110 or 112, to see whether the current time T matches an alarm time for inputting event information. It is to be appreciated that the structured sampling schema provides daily times (and hence alarms) for such collections. Also, ΔT can be periodic or determined by another algorithm where the algorithm determines ΔT dynamically so as to meet Table 7 requirements. If so, then in step 306 the processor 102 provides an alarm, such as an audio signal via speaker 148, visual signal via display 108, tactile signal (e.g. vibrations), email message, SMS, etc. to the patient 142. In step 308, after the patient 142 acknowledges the alarm via use of the user interface 146, insertion of a strip 140 into the strip reader 138, or after some set period of time, such as via expiration of a count down timer, the processor prompts the patient 142 for entry of the event information, via the questionnaire displayed on the display 108 by the processor 102.

In step 310, if the processor 102 fails to detect an entry via the user interface 146, after expiration of another count down timer e.g., 300 seconds (or in other embodiments the timer can range from few minutes to half an hour, and preferably 5 to 10 minutes), then the processor 102 in step 312 resets the alarm for a future time T which is still within the compliancy window of Table 7 for collecting the event information. If an entry was made and detected in step 310, such as placed into temporary memory 110 via the processor accepting input from the user interface 146, then in step 314 the processor 102 stores the entry in memory 112 in a manner discussed previously above.

If in step 304, the structured sampling schema in memory 110 or 112 does not have an alarm for the processor 102, then the processor 102 in step 316 will check for any triggered events, e.g. auto initiated via another running process of the meter 100 or patient initiated via the user interface 146. If none is detected, then the processor 102 loops back to step 302 and the processes of the event scheduler 300 repeat. If a trigger event is detected, then in step 318 the processor 102 checks whether an entry is needed for the triggered event, such as by doing a lookup in the profile file. If an entry is needed then the process goes to step 308, and if not, the process loops back to step 302 and repeats. It is to be appreciated that the scheduler 300 when executed by the processor 102 of the meter 100 indicates and consequently records a SMBG measurement and the associated event information (e.g., via running the questionnaire) in compliance with the measurement schema provided according to Table 7. Any unforeseen event is also enterable into memory 112 of the meter 100 at any time by the patient 142 via manually running the questionnaire on the meter 100 e.g. a triggered event in step 312.

Returning to FIG. 18, in step 204, the processor 102 checks to determine whether the bG data was collected in a compliant manner. Data compliant in step 204 means that rules and guidelines to collect data which were either programmatically or manually complied to by the user. In one embodiment, compliance check includes: checking to see whether the number of days in the bG data meets the minimum number needed to satisfy the nDay requirement (i.e., a predetermined period, which in one embodiment is >2 weeks if an HCP desires to use a few weeks of data to predict a future HbA1C and true mean bG in order to revise a patient's current therapy or behavior in order to try achieve their targeted goals, >80 days for a result having a CV <3%, or any amount of days therebetween for a snapshot); and checking to see whether a minimum number of the samples (nSample) collected at a requested sampling event per the collection schema (i.e., a predetermined amount, which in one preferred embodiment is >45, but in other embodiments may be some other amount that is reasonable for a patient's lifestyle and/or to address a desired need or question as determined by the HCP) also satisfy the sampling time window requirement (e.g., WinSize < 50 minutes). For example, in another embodiment, the structured sampling schema provided in memory is for a snapshot which provides a prediction of the probable HbA1c level and/or of the effect an action is having on a patient's HbA1C, via the change indicated by the estimated HbA1C. The structured sampling schema has the following parameters which the processor 102 checks the bG data in memory 112 as described above that resulted from a structured collection of seven spot measurements (before each meal, 2 hours after each meal, and before going to bed, for 3 consecutive days): nDays is at least 3 days, and the nSamples is at least 21 bG measurements with a WinSize <50 minutes. In one embodiment, the predetermined amount and period is read by the processor 102 from the structured sampling schema provided in memory, and in one embodiment, selected from a plurality of different structured sampling schema (e.g., focused or structured collection procedures). The result of the check on the collected bG data is either yes or no. In step 206, optionally, the processor 102 then checks for distribution of the time of bG samples with respect to the window center to see if there is a bias. If there is, then in step 208 a correction for the bias may be added to the data. For example, if the time for bG measurement with respect to targeted post-prandial average time is biased then the algorithm will systematically introduce delay in the future measurement of bG such as by alerting the patient latter with respect to the targeted measurement time or may be raise an additional alert for measurement at a subsequent latter time and thus over period of days remove bias from the average time of measurement.

In another embodiment an associated penalty in the precision of the estimated value may be flagged in step 208 such that a bias warning message is indicated with the provided results in step 214. After step 204, and optional step 206, if the bG data is compliant then the estimation processes Steps 210 and 212 are evoked. In step 210, the estimation process as mentioned previously above in reference to FIG. 7 bins together the data as per lifestyle as per event. The weighted mean bG is then determined by using the temporal weighting (harmonic weighting), whereby each of the weighted mean bG is then further time weighted by lifestyle related weight. The resulting value from step 210 is the estimate of the true mean bG value. Optionally, the estimated bG value is provided with an uncertainty window around the predicted value as is shown by FIGS. 16A-E. In step 212, the estimated HbA1C is then determined by solving the equation given in Table 7 using the estimated bG value from step 212, and the results then provided in step 214.

In another embodiment, an enhancement to the above model in Table 7 is to obtain an HbA1C value from an HbA1C assay, which can then be used as the patient specific intercept value c, instead of the give value of 0.5702. Such an embodiment is considered an estimated HbA1C with a one point calibration. In still a further embodiment, another enhancement to the above model in Table 7, would be to obtain HbA1C using an HbA1C assay at two different points in time. These HbA1C values can then be used to determine a patient specific intercept value c and slope m. In such an embodiment, the two HbA1C values from the HbA1C assay not vary by more than 0.5 % to provide a good reliable slope m, assuming the assays are high quality (i.e., CV<2%). In another embodiment, the process 200 may then request whether the protocol file used for collection by the scheduler 300 needs updating in step 216. If so the protocol file is updated in step 218 via e.g., accepting user input via the user interface 146, e.g., from the processor 102 re-running a setup questionnaire on the display 108, receiving protocol changes from the HCP computer 134 when connected to docking station 132, and combinations thereof. Afterwards, the process loops to step 202 and repeats.

In still other embodiments, collection step 202, along with the scheduler 300, is solely performed on the meter 100, wherein process steps 204-218 are performed on the HCP computer 134. In such an embodiment, the HCP computer 134 may also provide additional capabilities, such as using the collected bG data with other models to perform comparisons with the model results according to the present invention. For example, in one embodiment, the HCP could run the collected bG data through a HbA1C population based model derived from continuously monitored glucose data.

As previously mentioned above, in one embodiment the alerting and collecting by the processing of bG measurements and associated context of the bG measurement at the daily times and the events can be specified by the structured sampling schema that is stored in memory. In one embodiment, the daily times specified by the structured sampling schema are post-prandial times. In another embodiment, the daily times specified by the structured sampling schema are three post-prandial times and another time. In still another embodiment, the events specified by the structured sampling schema is a specific time with respect to start of a meal. In yet another embodiment, one of the events specified by the structured sampling schema is an aspect of glucose behavior related to the estimated true mean bG value, which in one embodiment, the aspect is a bG mean to peak value. In still another embodiment, the daily times specified by the structured sampling schema are at about 140 to about 240 minutes after a meal time. In a further embodiment, the daily times and the events specified by a structured sampling schema are tailored to a daily lifestyle pattern of the patient. In yet another embodiment, the daily times specified by the structured sampling schema range from about 140 to about 240 minutes after a meal time in accordance with the daily lifestyle pattern of the patient.

In another embodiment, the processor 102 is further programmed to weigh a bG measurement if collection of the bG measurement was within a time interval from the daily times specified by the structured sampling schema, whereby in one embodiment the time interval is at most ±50 minutes. It is to be appreciated that the time interval also captures the information of whether the measurements, which are typically performed by the patient at random times around a recommended time, are falling within or outside the time interval. Such information may be used by the processor 102 to evaluate whether the patient's lifestyle has been captured appropriately as reflected in the structured sampling schema and/or whether the patient requires training such as, for example, if a threshold number of measurement within the time interval is not meet over a period of time. For example, in one embodiment, if such a threshold number of measurements is not achieved, the processor 102 provides a message on the display 108 indicating a collection problem and can provide a recommendation, such as ways to improve collection compliancy. In still another embodiment, the processor 102 is further programmed to determine the estimated true mean bG value and the estimate HbA1C value from the weighted measurements of the collected bG measurements if a predetermined amount of the bG measurements per each of the daily times and the events has been collected. In one preferred embodiment, the predetermined amount is at least 80 days, and in another embodiment at least 60 days. In still another embodiment, the processor is further programmed to determine the estimated true mean bG value and the estimate HbA1C value from the weighted measurements of the collected bG measurements if the predetermined amount of the bG measurements per each of the daily times and the events has been collected, and if the collection of the bG measurements occurred within a predetermined period of at least 2 weeks. In still other embodiment, other predetermined periods may be used, such as at least 3 days in order to provide a prediction of a probably HbA1c level and/or of the effect an action (i.e., exercising, dieting, taking a prescription, sleeping, changing a habit(s), a change in physiological and/or psychological condition(s), etc.) is having on a patient's HbA1C, via the change indicated by the estimated HbA1C.

In summary, the embodiments of the present invention addresses the stated problems noted in the background of the invention. Some of the noted advantages of the embodiments of the present invention include, for example and not limited thereto, no retuning of model parameters needed as the prediction model (solution) is robust and more accurate then the other solutions which are purely based on SMBG measurements that fail to consider the sampling context; no model parameter update needed; and that measurement protocol/rule sets are defined. Additionally, patient specific solutions are provided as the method is customizable to a patient's lifestyle which is extracted from patient data collected over a period of time or collected via an interview with the patient, and accounted for by lifestyle related weighting.

It is to be appreciated that the prediction model is physiology based, wherein fundamental physiology behind HbA1C formation is used to deduce the impact of bG on HbA1C. The fixed physiology captures the essence of HbA1C and the resulting HbA1C is then a measure of overall glycemia as per a standard biological process. This means that the patient specific physiology is discounted. Using such a metric which is independent of patient specific physiology is useful in helping an HCP to better evaluate glycemic level using a consistent basis. With the embodiments of the present invention, one can use a recent data set to predict a future HbA1C which will indicate the impact of the current lifestyle of the patient if maintained and continued.

Having described the disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

**Claims**

1. A computer-implemented method for providing both an estimated true mean blood glucose value and estimated glycated hemoglobin (HbA1C) value from spot blood glucose (bG) measurements of a patient comprising:

   collecting both bG measurements and associated context of the bG measurement at daily times and events specified by a structured sampling schema;
   weighting each of the collected bG measurements based on the associated context;
   wherein the weighting is based on both duration between meals and age of bG measurements;
   determining the estimated true mean bG value and the estimate HbA1C value from the weighted measurements of the collected bG measurements; and
   providing the estimated true mean bG and the estimate HbA1C values.

2. The method according to claim 1 wherein the daily times specified by the structured sampling schema are post-prandial times.

3. The method according to claim 1 wherein the collected bG measurements are collected over 3 consecutive days to provide a prediction of a probably HbA1c level and/or of the effect an action is having on HbA1C of the patient.

4. The method according to claim 1 wherein one of the events specified by the structured sampling schema is a specific time with respect to start of a meal.

5. The method according to claim 1 wherein the daily times specified by the structured sampling schema are at about 140 to about 240 minutes after a meal time.

6. The method according to claim 1 wherein the daily times and the events specified by a structured sampling schema are tailored to a daily lifestyle pattern of the patient.

7. The method according to claim 1 wherein the structured sampling schema requires collection of the bG measurement within a time interval from the daily times specified by the structured sampling schema in order for a bG measurement to be weighted.

8. The method according to claim 1 wherein the structured sampling schema requires collection of a predetermined amount of the bG measurements per each of the daily times and the events before the method can provide the estimated true mean bG and the estimate HbA1C values.

9. The method according to claim 1 wherein the structured sampling schema requires collection of the bG measurements within a predetermined period before the method can provide the estimated true mean bG and the estimate HbA1C values.

10. The method according to claim 1 wherein the weighting is based on harmonic temporal weighting.

11. The method according to claim 1 wherein the collected bG measurements older than 120 days have a weight of zero.

12. The method according to claim 1 wherein the estimated true mean bG value ($\overline{bG}$) is determined by averaging mean bG values for the daily times, each being defined as:

$$\overline{bG} = \frac{T^{FASTING}}{24}\,\overline{bG}_{FASTING} + \frac{T^{BF}}{24}\,\overline{bG}^{BF} + \frac{T^{LU}}{24}\,\overline{bG}^{LU} + \frac{T^{SU}}{24}\,\overline{bG}^{SU},$$

where $\overline{bG}_{FASTING}$ is a bG measurement specified in the structured sampling schema at a time after a fasting time, $\overline{bG}^{BF}$ is a bG measurement specified in the structured sampling schema at a post-prandial time after a breakfast time, $\overline{bG}^{LU}$ is a bG measurement specified in the structured sampling schema at a post-prandial time after a lunch time, $\overline{bG}^{SU}$ is a bG measurement specified in the structured sampling schema at a post-prandial time after a supper time, and each respective T represents duration of time between consecutive meal events.

13. A system for providing both an estimated true mean blood glucose value and estimated glycated hemoglobin (HbA1C) value from spot blood glucose (bG) measurements comprising:

a display;
memory; and
a processor programmed:

to collect both bG measurements and associated context of the bG measurement at daily times and events specified by a structured sampling schema provided in memory;
to weight each of the collected bG measurements based on the associated context;
wherein the weighting is based on both duration between meals and age of bG measurements;
to determine the estimated true mean bG value and the estimate HbA1C value from the weighted measurements of the collected bG measurements; and
to provide the estimated true mean bG and the estimate HbA1C values to the display.

14. A system according to claim 13 comprising:

a blood glucose monitoring meter having memory and a first processor programmed to collect both bG measurements and associated context of the bG measurement at daily times specified by a structured sampling schema provided in the memory; and
a computer having a display, memory and a second processor programmed:

to receive the collected bG measurements and associated context from the meter;
to weight each of the collected bG measurements based on the associated context;
to determine the estimated true mean bG value and the estimate HbA1C value from the weighted measurements of the collected bG measurements; and
to provide the estimated true mean bG and the estimate HbA1C values to the display.

**Patentansprüche**

1. Computerimplementiertes Verfahren zur Bereitstellung eines geschätzten wahren Blutglukosemittelwertes und eines geschätzten Glykohämoglobin-Wertes (HbA1C) aus Spot-Blutglukose-(bG)-Messungen von einem Patienten, das umfasst:

Erfassen von bG-Messungen und des zugehörigen Kontextes der bG-Messung zu täglichen Zeiten und Anlässen, die von einem strukturierten Probenahmeschema vorgegeben sind;
Gewichten jeder der erfassten bG-Messungen basierend auf dem zugehörigen Kontext;
wobei das Gewichten auf der Dauer zwischen Mahlzeiten und dem Alter der bG-Messungen basiert;
Bestimmen des geschätzten wahren bG-Mittelwertes und des geschätzten HbA1C-Wertes aus den gewichteten Messungen der erfassten bG-Messungen; und
Bereitstellen des geschätzten wahren bG-Mittelwertes und des geschätzten HbA1C-Wertes.

2. Verfahren nach Anspruch 1, wobei die von dem strukturierten Probenahmeschema vorgegebenen täglichen Zeiten postprandiale Zeiten sind.

3. Verfahren nach Anspruch 1, wobei die erfassten bG-Messungen über 3 aufeinanderfolgende Tage erfasst werden, um eine Vorhersage eines wahrscheinlichen HbA1c-Spiegels und/oder der Wirkung, die eine Maßnahme auf den HbA1C des Patienten hat, bereitzustellen.

4. Verfahren nach Anspruch 1, wobei einer der von dem strukturierten Probenahmeschema vorgegebenen Anlässe eine bezogen auf den Beginn einer Mahlzeit spezifische Zeit ist.

5. Verfahren nach Anspruch 1, wobei die von dem strukturierten Probenahmeschema vorgegebenen täglichen Zeiten bei etwa 140 bis etwa 240 Minuten nach einer Mahlzeit liegen.

6. Verfahren nach Anspruch 1, wobei die von einem strukturierten Probenahmeschema vorgegebenen täglichen Zeiten und Anlässe auf eine tägliche Lebensführung des Patienten zugeschnitten sind.

7. Verfahren nach Anspruch 1, wobei das strukturierte Probenahmeschema die Erfassung der bG-Messung innerhalb eines Zeitintervalls aus den von dem strukturierten Probenahmeschema vorgegebenen täglichen Zeiten erfordert, damit eine bG-Messung gewichtet werden kann.

8. Verfahren nach Anspruch 1, wobei das strukturierte Probenahmeschema die Erfassung einer vorbestimmten Menge der bG-Messungen für jede/jeden der täglichen Zeiten und Anlässe erfordert, bevor das Verfahren den geschätzten wahren bG-Mittelwert und den geschätzten HbA1C-Wert bereitstellen kann.

9. Verfahren nach Anspruch 1, wobei das strukturierte Probenahmeschema die Erfassung der bG-Messungen innerhalb eines vorbestimmten Zeitraumes erfordert, bevor das Verfahren den geschätzten wahren bG-Mittelwert und den geschätzten HbA1C-Wert bereitstellen kann.

10. Verfahren nach Anspruch 1, wobei das Gewichten auf harmonischem zeitlichem Gewichten basiert.

11. Verfahren nach Anspruch 1, wobei erfasste bG-Messungen, die älter sind als 120 Tage, eine Wertigkeit von null haben.

12. Verfahren nach Anspruch 1, wobei der geschätzte wahre bG-Mittelwert ($\overline{bG}$) bestimmt wird, indem bG-Mittelwerte für die täglichen Zeiten gemittelt werden, wobei jeder definiert ist als:

$$\overline{bG} = \frac{T^{N\ddot{U}CHTERN}}{24}\overline{bG}_{N\ddot{U}CHTERN} + \frac{T^{BF}}{24}\overline{bG}^{BF} + \frac{T^{LU}}{24}\overline{bG}^{LU} + \frac{T^{SU}}{24}\overline{bG}^{SU},$$

wobei $\overline{bG}_{N\ddot{U}CHTERN}$ eine in dem strukturierten Probenahmeschema vorgegebene bG-Messung zu einem Zeitpunkt nach einer Nüchternzeit ist, $\overline{bG}^{BF}$ eine in dem strukturierten Probenahmeschema vorgegebene bG-Messung zu einem postprandialen Zeitpunkt nach einer Frühstückszeit ist, $\overline{bG}^{LU}$ eine in dem strukturierten Probenahmeschema vorgegebene bG-Messung zu einem postprandialen Zeitpunkt nach einer Mittagessenzeit ist, $\overline{bG}^{SU}$ eine in dem strukturierten Probenahmeschema vorgegebene bG-Messung zu einem postprandialen Zeitpunkt nach einer Abendessenzeit ist und jedes entsprechende T die Zeitdauer zwischen aufeinanderfolgenden Mahlzeitanlässen darstellt.

13. System zur Bereitstellung eines geschätzten wahren Blutglukosemittelwertes und eines geschätzten Glykohämoglobin-Wertes (HbA1C) aus Spot-Blutglukose (bG)-Messungen, das umfasst:

eine Anzeige;
Speicher; und
einen Prozessor, der so programmiert ist, dass er:

bG-Messungen und zugehörigen Kontext der bG-Messung zu täglichen Zeiten und Anlässen, die von einem in dem Speicher bereitgestellten strukturierten Probenahmeschema vorgegeben sind, erfasst;
jede der erfassten bG-Messungen basierend auf dem zugehörigen Kontext gewichtet;
wobei das Gewichten auf der Dauer zwischen Mahlzeiten und dem Alter der bG-Messungen basiert;
den geschätzten wahren bG-Mittelwert und den geschätzten HbA1C-Wert aus den gewichteten Messungen der erfassten bG-Messungen bestimmt; und
den geschätzten wahren bG-Mittelwert und den geschätzten HbA1C-Wert auf der Anzeige bereitstellt.

**14.** System nach Anspruch 13, umfassend:

ein Blutglukose-Überwachungsmessgerät mit Speicher und einem ersten Prozessor, der so programmiert ist, dass er bG-Messungen und zugehörigen Kontext der bG-Messung zu täglichen Zeiten erfasst, die von einem in dem Speicher bereitgestellten strukturierten Probenahmeschema vorgegeben sind; und
einen Computer mit einer Anzeige, Speicher und einem zweiten Prozessor, der so programmiert ist, dass er:

die erfassten bG-Messungen und zugehörigen Kontext von dem Messgerät empfängt;
jede der erfassten bG-Messungen basierend auf dem zugehörigen Kontext gewichtet;
den geschätzten wahren bG-Mittelwert und den geschätzten HbA1C-Wert anhand der gewichteten Messungen der erfassten bG-Messungen bestimmt; und
den geschätzten wahren bG-Mittelwert und den geschätzten HbA1C-Wert auf der Anzeige bereitstellt.

## Revendications

**1.** Procédé mis en oeuvre par ordinateur fournissant à la fois un taux de glycémie moyen réel estimé et un taux d'hémoglobine glycosylée (HbA1C) estimé à partir de mesures ponctuelles de la glycémie (bG) d'un patient comprenant :

la collecte à la fois des mesures de bG et du contexte associé de la mesure de bG à des temps quotidiens et des événements spécifiés par un schéma d'échantillonnage structuré ;
la pondération de chacune des mesures de bG collectées sur la base du contexte associé ;
la pondération se basant à la fois sur la durée entre les repas et l'ancienneté des mesures de bG ;
la détermination du taux de bG moyen réel estimé et du taux d'HbA1C estimé à partir des mesures pondérées des mesures de bG collectées ; et
la fourniture des taux de bG moyen réel estimé et d'HbA1C estimé.

**2.** Procédé selon la revendication 1 dans lequel les temps quotidiens spécifiés par le schéma d'échantillonnage structuré sont des temps postprandiaux.

**3.** Procédé selon la revendication 1 dans lequel les mesures de bG collectées sont collectées sur 3 jours consécutifs pour fournir une prédiction d'un taux d'HbA1C probable et/ou de l'effet qu'une action exerce sur l'HbA1C du patient.

**4.** Procédé selon la revendication 1 dans lequel l'un des événements spécifiés par le schéma d'échantillonnage structuré est un temps spécifique par rapport au début d'un repas.

**5.** Procédé selon la revendication 1 dans lequel les temps quotidiens spécifiés par le schéma d'échantillonnage structuré sont d'environ 140 à environ 240 minutes après un repas.

**6.** Procédé selon la revendication 1 dans lequel les temps quotidiens et les événements spécifiés par un schéma d'échantillonnage structuré sont adaptés à un mode de vie quotidien du patient.

**7.** Procédé selon la revendication 1 dans lequel le schéma d'échantillonnage structuré nécessite la collecte de la mesure de bG dans un intervalle de temps à partir des temps quotidiens spécifiés par le schéma d'échantillonnage structuré pour qu'une mesure de bG soit pondérée.

8. Procédé selon la revendication 1 dans lequel le schéma d'échantillonnage structuré nécessite la collecte d'une quantité prédéterminée des mesures de bG pour chacun des temps quotidiens et des événements avant que le procédé puisse fournir les taux de bG moyen réel estimé et d'HbA1C estimé.

9. Procédé selon la revendication 1 dans lequel le schéma d'échantillonnage structuré nécessite la collecte des mesures de bG dans une période prédéterminée avant que le procédé puisse fournir les taux de bG moyen réel estimé et d'HbA1C estimé.

10. Procédé selon la revendication 1 dans lequel la pondération se base sur une pondération temporelle harmonique.

11. Procédé selon la revendication 1 dans lequel les mesures de bG collectées anciennes de plus de 120 jours ont un poids de zéro.

12. Procédé selon la revendication 1 dans lequel le taux de bG moyen réel estimé ($\overline{bG}$) est déterminé en calculant la moyenne des valeurs de bG moyennes pour les temps quotidiens, chacune étant définie comme :

$$\overline{bG} = \frac{T^{JE\hat{U}NE}}{24}\overline{bG}_{JE\hat{U}NE} + \frac{T^{BF}}{24}\overline{bG}^{BF} + \frac{T^{LU}}{24}\overline{bG}^{LU} + \frac{T^{SU}}{24}\overline{bG}^{SU},$$

où $\overline{bG}_{JE\hat{U}NE}$ est une mesure de $bG$ spécifiée dans le schéma d'échantillonnage structuré à un temps qui suit un jeûne, $\overline{bG}^{BF}$ est une mesure de bG spécifiée dans le schéma d'échantillonnage structuré à un temps postprandial qui suit un petit déjeuner, $\overline{bG}^{LU}$ est une mesure de bG spécifiée dans le schéma d'échantillonnage structuré à un temps postprandial après un déjeuner, $\overline{bG}^{SU}$ est une mesure de bG spécifiée dans le schéma d'échantillonnage structuré à un temps postprandial qui suit un dîner, et chaque T respectif représente la durée entre deux événements de repas consécutifs.

13. Système fournissant à la fois un taux de glycémie moyen réel estimé et un taux d'hémoglobine glycosylée (HbA1C) estimé à partir de mesures ponctuelles de la glycémie (bG) comprenant :

un dispositif d'affichage ;
une mémoire ; et
un processeur programmé :

pour collecter à la fois les mesures de bG et le contexte associé de la mesure de bG à des temps quotidiens et des événements spécifiés par un schéma d'échantillonnage structuré fourni dans la mémoire ;
pour pondérer chacune des mesures de bG collectées sur la base du contexte associé ;
la pondération se basant à la fois sur la durée entre les repas et l'ancienneté des mesures de bG ;
pour déterminer le taux de bG moyen réel estimé et le taux d'HbA1C estimé à partir des mesures pondérées des mesures de bG collectées ; et
pour fournir les taux de bG moyen réel estimé et d'HbA1C estimé au dispositif d'affichage.

14. Système selon la revendication 13 comprenant :

un lecteur de glycémie ayant une mémoire et un premier processeur programmé pour collecter à la fois les mesures de bG et le contexte associé de la mesure de bG à des temps quotidiens spécifiés par un schéma d'échantillonnage structuré fourni dans la mémoire ; et
un ordinateur ayant un dispositif d'affichage, une mémoire et un second processeur programmé :

pour recevoir les mesures de bG collectées et le contexte associé à partir du lecteur ;
pour pondérer chacune des mesures de bG collectées sur la base du contexte associé ;
pour déterminer le taux de bG moyen réel estimé et le taux d'HbA1C estimé à partir des mesures pondérées des mesures de bG collectées ; et
pour fournir les taux de bG moyen réel estimé et d'HbA1C estimé au dispositif d'affichage.

EP 2 445 394 B1

| TIME | COHORT - 1 $C_1$ | | | COHORT - 2 $C_2$ | | | ... ... | | | COHORT - n $C_n$ | | |
|---|---|---|---|---|---|---|---|---|---|---|---|---|
| | HB | $HB_A$ | $HB_{AIC}$ | HB | $HB_A$ | $HB_{AIC}$ | HB | $HB_A$ | $HB_{AIC}$ | HB | $HB_A$ | $HB_{AIC}$ |
| $t_1$ | | ... | | | ... | | | ... | | | ... | |
| ... | | ... | | | ... | | | ... | | | ... | |

FIG. 1

$T_{BF}$   $T_{LU}$   $T_{SU}$   $T_{ON}$

OVERNIGHT   BREAKFAST   LUNCH   SUPPER   OVERNIGHT

0:00   4:00   8:00   12:00   16:00   20:00   24:00

CONTINUES INTO NEXT DAY

START OF DAY

END OF DAY

FIG. 5

FIG. 2

FIG. 3

A1C versus Mean bG Excursion

Legend:
— Simulated Mean bG (+ve)
— Simulated Mean bG (-ve)
—△— Ref A1C to mean bG

A1C %

Mean Excursion [mg/dL]

FIG. 4

FIG. 6

FIG. 7

FIG. 8

EP 2 445 394 B1

FIG. 9

FIG. 10

FIG. 11

FIG. 12

EP 2 445 394 B1

Harmonic LS + Repetition Set=1

FIG. 13

Nos of Samples/Visit-Event []

Mean Square Error

Legend:
● Visit Period 50
□ Visit Period 60
◇ Visit Period 70
▽ Visit Period 80
✳ Visit Period 90

Harmonic LS + Repetition Set=5

FIG. 14

FIG. 15A · FIG. 15B · FIG. 15C · FIG. 15D · FIG. 15E

FIG.16C

FIG.16B

FIG.16E

FIG.16A

FIG.16D

FIG. 17

*200*

FIG. 18

FIG. 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 643338 A
- US 297733 A

- US 12119201 B

**Non-patent literature cited in the description**

- **MORTENSEN, H. B.** Glycated hemoglobin. Reaction and biokinetic studies. Clinical application of hemoglobin A1c in the assessment of metabolic control in children with diabetes mellitus. *Danish medical bulletin,* 1985, vol. 32 (6), 309-328